(19) Europäisches Patentamt / European Patent Office / Office européen des brevets

(11) **EP 2 901 984 A1**

(12) **EUROPEAN PATENT APPLICATION**
published in accordance with Art. 153(4) EPC

(43) Date of publication:
05.08.2015 Bulletin 2015/32

(51) Int Cl.:
*A61F 13/15* $^{(2006.01)}$        *A61F 13/53* $^{(2006.01)}$

(21) Application number: 13841665.6

(22) Date of filing: 20.08.2013

(86) International application number:
PCT/JP2013/072161

(87) International publication number:
WO 2014/050366 (03.04.2014 Gazette 2014/14)

(84) Designated Contracting States:
AL AT BE BG CH CY CZ DE DK EE ES FI FR GB
GR HR HU IE IS IT LI LT LU LV MC MK MT NL NO
PL PT RO RS SE SI SK SM TR
Designated Extension States:
BA ME

(30) Priority: 30.09.2012 JP 2012218977
25.06.2013 JP 2013133048

(71) Applicant: Unicharm Corporation
Shikokuchuo-shi, Ehime 799-0111 (JP)

(72) Inventors:
• UDA, Masashi
  Kanonji-shi
  Kagawa 769-1602 (JP)
• MARUYAMA, Takashi
  Kanonji-shi
  Kagawa 769-1602 (JP)

(74) Representative: Eke, Philippa Dianne
Saunders & Dolleymore LLP
9 Rickmansworth Road
Watford Hertfordshire WD18 0JU (GB)

(54) **ABSORBENT ARTICLE**

(57) The present invention addresses the problem of, in an absorbent article, preventing the leakage of fiber constituting an absorbent core from a non-covered region through a liquid-permeable layer, said leakage possibly occurring when the strength of the absorbent core is decreased due to liquid absorption. To solve this problem, provided is a sanitary napkin (1) which comprises a top sheet (2), a back sheet (3) and an absorbent core (4) that is interposed between the top sheet (2) and the back sheet (3), wherein: the absorbent core (4), which involves a non-covered region where the constituting fiber is exposed on the surface and in contact directly with the top sheet (2), contains a thermoplastic resin fiber, said thermoplastic resin fiber containing as a monomer component an unsaturated carboxylic acid, an unsaturated carboxylic acid anhydride or a mixture thereof, at a mixing ratio by mass to a cellulose-based water absorbent fiber of 1/9 or more; and the wet fiber fall-off rate of the absorbent core (4) is controlled to 13.3% or less.

FIG. 1

EP 2 901 984 A1

**Description**

Technical Field

The present invention relates to an absorbent article.

Background Art

As an absorbent article, one comprising a liquid-permeable top sheet, a liquid-impermeable back sheet, and a liquid-retentive absorbent core there has been known, wherein the top sheet covers the skin side surface of the absorbent core and is directly joined to the skin side surface (i.e., there is no other layer disposed between the top sheet and the absorbent core) (Patent Literature 1).

As a thermal adhesive conjugate fiber for air-laid nonwoven fabrics, core-sheath type conjugate fibers have been known, comprising as a sheath component a modified polyolefin having a vinyl monomer graft-polymerized thereto, wherein the vinyl monomer comprising a unsaturated carboxylic acid or unsaturated carboxylic acid anhydride, and as a core component a resin having a melting point higher than that of the modified polyolefin (Patent Literatures 2 and 3).

Citation List

Patent Literature

Patent Literature 1: Japanese Unexamined Patent Publication No. 2012-075637
Patent Literature 2: Japanese Patent No. 4221849
Patent Literature 3: Japanese Unexamined Patent Publication No. 2004-270041

Summary of Invention

Problems to be Solved by the Invention

The absorbent core disclosed in Patent Literature 1 has a non-covered region where the constituent fibers thereof are exposed to the surface so that the constituent fibers directly contact with the top sheet. Accordingly, the absorbent article described in Patent Literature 1 is liable to cause leakage of the constituent fibers of the absorbent core through the top sheet from the non-covered region due to collapse of the absorbent core, interfacial delaminating between the top sheet and the absorbent core, etc., when the strength of the absorbent core is decreased due to liquid absorption.

With respect to the core-sheath type conjugate fibers described in Patent Literatures 2 and 3, it has been known that the core-sheath type conjugate fibers have good adhesive properties to cellulose-based fibers, whereas its availability as a component of the absorbent core has been unknown.

Therefore, the present invention is directed to provide an absorbent article comprising an absorbent core comprising cellulose-based water absorbent fibers and thermoplastic resin fibers comprising as a monomer component a unsaturated carboxylic acid, a unsaturated carboxylic acid anhydride, or a mixture thereof, and is capable of preventing the constituent fibers of the absorbent core from leaking through the liquid-permeable layer from a non-covered region, which may be caused when the strength of the absorbent core is decreased due to liquid absorption.

Solution to Problem

In order to solve the above problems, the present invention provides an absorbent article comprising a liquid-permeable layer, a liquid-impermeable layer, and an absorbent core disposed between the liquid-permeable layer and the liquid-impermeable layer, wherein the absorbent core comprises as constituent fibers cellulose-based water absorbent fibers and thermoplastic resin fibers comprising as a monomer component a unsaturated carboxylic acid, a unsaturated carboxylic acid anhydride, or a mixture thereof, wherein the absorbent core has a non-covered region where the constituent fibers thereof are exposed to a surface so that the constituent fibers directly contact with the liquid-permeable layer, wherein the weight ratio of the thermoplastic resin fibers to the water absorbent fibers in the absorbent core is 1/9 or more, and wherein the absorbent core has a fiber falling-off rate in wet state of 13.3% or less.

Effects of the Invention

According to the present invention, an absorbent article comprising an absorbent core comprising as a monomer component a unsaturated carboxylic acid, a unsaturated carboxylic acid anhydride, or a mixture thereof, and capable of preventing the constituent fibers of the absorbent core from leaking through the top sheet from a non-covered region, which may be caused when the strength of the absorbent core is decreased due to liquid absorption is provided.

Brief Description of Drawings

[Fig. 1] Fig. 1 is a partially cutaway plan view of a sanitary napkin according to an embodiment of the present invention.
[Fig. 2] Fig. 2 is an A-A line cross-sectional view of Fig. 1.
[Fig. 3] Fig. 3 is a diagram showing a manufacturing process of the sanitary napkin according to an embodiment of the present invention.
[Fig. 4] Figs. 4 (a) and (b) are diagrams showing the SJ belt press machine used in the examples.

Description of Embodiments

An absorbent article of the present invention will be explained below.
The absorbent article according to Embodiment 1 comprises a liquid-permeable layer, a liquid-impermeable layer, and an absorbent core disposed between the liquid-permeable layer and the liquid-impermeable layer, wherein the absorbent core comprises as constituent fibers cellulose-based water absorbent fibers (hereinafter also abbreviated as "water absorbent fibers") and thermoplastic resin fibers (hereinafter also abbreviated as "thermoplastic resin fibers") comprising as a monomer component a unsaturated carboxylic acid, a unsaturated carboxylic acid anhydride, or a mixture thereof, wherein the absorbent core has a non-covered region where the constituent fibers thereof are exposed to a surface so that the constituent fibers directly contact with the liquid-permeable layer, wherein the weight ratio of the thermoplastic resin fibers to the water absorbent fibers in the absorbent core is 1/9 or more, and wherein the absorbent core has a fiber falling-off rate in wet state of 13.3% or less.
The absorbent article according to Embodiment 1 can prevent the constituent fibers of the absorbent core from leaking through the liquid-permeable layer from a non-covered region, which may be caused when the strength of the absorbent core is decreased due to liquid absorption. Therefore, the absorbent article according to Embodiment 1 is less likely to give the wearer uncomfortable feeling due to fiber leakage.
The higher the percentage of the non-covered region to the interface between the liquid-permeable layer and the absorbent core, the greater the possibility to leak the constituent fibers of the absorbent core through the liquid-permeable layer from the non-covered region. Therefore, the fiber leakage prevention effect of the absorbent article according to Embodiment 1 is more remarkable with the increase in the proportion of the non-covered region to the interface between the liquid-permeable layer and the absorbent core. From such a view point, the proportion of the non-covered region to the interface between the absorbent core and the liquid-permeable layer is preferably 10% or more, and more preferably 30% or more. The upper limit is 100%.
In a preferred embodiment (Embodiment 2) of the absorbent article according to Embodiment 1, the absorbent core has a fiber falling-off rate in dry state of 2.8% or less.
The absorbent article according to Embodiment 2 maintains a sufficient strength before and after liquid absorption (i.e., not only in dry state but also in wet state), and thereby achieves a fiber falling-off rate in dry state of 2.8% or less for the absorbent core and a fiber falling-off rate in wet state of 13.3% or less for the absorbent core. Accordingly, the absorbent article according to Embodiment 2 can effectively prevent the absorbent core from collapsing and can effectively prevent the constituent fibers of the absorbent core from leaking through the liquid-permeable layer from the non-covered region resulting from the collapsing of the absorbent core, even if the strength of the absorbent core is decreased upon liquid absorption. Therefore, the absorbent article according to Embodiment 2 is less likely to give uncomfortable feeling to the wearer due to fiber leakage. The requirement that the weight ratio of the thermoplastic resin fibers to water absorbent fibers is 1/9 or more is an essential requirement to achieve a fiber falling-off rate in dry state of 2.8% or less for the absorbent core and a fiber falling-off rate in wet state of 13.3% or less for the absorbent core.
In a preferred embodiment (Embodiment 3) of the absorbent article according to Embodiment 1 or 2, the absorbent core contains a superabsorbent polymer (SAP) particles, and the absorbent core has a SAP particle falling-off rate in dry state of 14.3% or less.
The absorbent article according to Embodiment 3 can prevent the SAP particle of the absorbent core from leaking through the liquid-permeable layer from the non-covered region, which is likely to occur before liquid absorption. Therefore, the absorbent article according to Embodiment 3 is less likely to give uncomfortable feeling to the wearer due to fiber leakage. As with the constituent fibers of the absorbent core, the higher the percentage of the non-covered region to the interface between the liquid-permeable layer and the absorbent core, the greater the possibility to leak the constituent fibers of the absorbent core from the non-covered region through the liquid-permeable layer. Thus, the SAP particle leakage prevention effect of the absorbent article according to Embodiment 3 is more remarkable with the increase in the proportion of the non-covered region to the interface between the liquid-permeable layer and the absorbent core. From such a view point, the proportion of the non-covered region to the interface between the liquid-permeable layer and the absorbent core is preferably 10% or more, and more preferably 30% or more. The upper limit is 100%.

In a preferred embodiment (Embodiment 4) of the absorbent article according to any of Embodiments 1 to 3, the weight ratio of the thermoplastic resin fibers to the water absorbent fibers in the absorbent core is 1/9 to 5/5.

In the absorbent article according to Embodiment 4, the upper limit of 5/5 has been determined in view of the liquid absorbing properties of the absorbent core, and the absorbent core has a sufficient strength (particularly, wet strength after liquid absorption) and sufficient liquid absorbing properties when the weight ratio of the thermoplastic resin fibers to the water absorbent fibers is 1/9 to 5/5.

In a preferred embodiment (Embodiment 5) of the absorbent article according to Embodiment 4, the absorbent core has a fiber falling-off rate in dry state of 0.9 to 2.8%, and the absorbent core has a fiber falling-off rate in wet state of 1.0 to 13.3%.

The absorbent article according to Embodiment 5 maintains a sufficient strength before and after liquid absorption (i.e., not only in dry state but also in wet state), and thereby achieves a fiber falling-off rate in dry state of 0.9 to 2.8% for the absorbent core and a fiber falling-off rate in wet state of 1.0 to 13.3% for the absorbent core. Accordingly, the absorbent article according to Embodiment 5 can effectively prevent the absorbent core from collapsing and can effectively prevent the constituent fibers of the absorbent core from leaking through the liquid-permeable layer from the non-covered region resulting from the collapse of the absorbent core, even if the strength of the absorbent core is decreased due to liquid absorption. The weight ratio of the thermoplastic resin fibers to water absorbent fibers of 1/9 to 5/5 is an essential condition to achieve a fiber falling-off rate in dry state of 0.9% to 2.8% for the absorbent core and a fiber falling-off rate in wet state of 1.0 to 13.3% for the absorbent core.

In a preferred embodiment (Embodiment 6) of the absorbent article according to Embodiment 4 or 5, the absorbent core has a fiber density of 0.06 to 0.14 $g/cm^3$. If the absorbent core has a fiber density of 0.06 to 0.14 $g/cm^3$ when the weight ratio of the thermoplastic resin fibers to the water absorbent fibers in the absorbent core is 1/9 to 5/5, sufficient liquid absorbing properties can be imparted to the absorbent core.

In a preferred embodiment (Embodiment 7) of the absorbent article according to Embodiment 6, the water absorbent core is obtained by ejecting a high-pressure steam to a mixed material comprising cellulose-based water absorbent fibers and thermoplastic resin fibers to densify the mixed material. In the absorbent article according to Embodiment 7, the fiber density of the absorbent core is adjusted to a desired range by densification by means of ejecting of a high-pressure steam. Upon ejecting of a high-pressure steam to the mixed material, the steam is penetrated into the mixed material, thereby cleaving hydrogen bonds (for example, hydrogen bonds among water absorbent fibers, hydrogen bonds among thermoplastic resin fibers, hydrogen bonds among water absorbent fibers-thermoplastic resin fibers, etc.) to soften the mixed material. Accordingly, the pressure required for the densification is decreased, and the density of the softened mixed material can be easily adjusted. Reforming of hydrogen bonds by drying the mixed material having an adjusted density suppresses the elastic recovery of fibers (increase in bulkiness), thereby retaining the fiber density of the absorbent core within a specific range. Embodiment 7 is particularly preferred when an unsaturated carboxylic acid anhydride (for example, maleic anhydride or derivatives thereof) is contained in the thermoplastic resin fibers as a monomer component. If the unsaturated carboxylic acid anhydride groups contained in the thermoplastic resin fibers are converted to unsaturated carboxylic acid groups by the reaction with water vapor, the number of oxygen atoms capable of forming hydrogen bonds is increased, and therefore the elastic recovery (increase in bulkiness) of the densified fibers is suppressed effectively. Further, the mixed material may contain SAP particles, if desired (for example, in case of Embodiment 3), in addition to the cellulose-based water absorbent fibers and thermoplastic resin fibers.

In a preferred embodiment (Embodiment 8) of the absorbent article according to Embodiment 6 or 7, the absorbent core has a fiber basis weight of 40 to 900 $g/m^2$. If the fiber basis weight is less than 40 $g/m^2$, the fiber amount of the thermoplastic resin fiber is insufficient, and the strength (particularly the wet strength after liquid absorption) of the absorbent core may possibly be decreased, whereas if the fiber basis weight is more than 900 $g/m^2$, the fiber amount of the thermoplastic resin fibers is excessive, and the rigidity of the absorbent core may possibly be too high.

In a preferred embodiment (Embodiment 9) of the absorbent article according to any one of Embodiments 4 to 8, the absorbent article further comprises a joint section at which the liquid-permeable layer and the absorbent core are joined with each other, wherein the joint section has a joining strength in dry state of 1.53 to 7.65 N/25 mm, and the joint section has a joining strength in wet state of 0.95 to 4.34 N/25 mm.

In the absorbent article according to Embodiment 9, the absorbent core maintains a sufficient strength before and after liquid absorption (i.e., not only in dry state but also in wet state), and thereby achieves a joining strength in dry state of 1.53 to 7.65 N/25 mm and a joining strength in wet state of 0.95 to 4.34 N/25 mm. Therefore, the absorbent article according to Embodiment 9 can effectively prevent the interfacial delamination between the liquid-permeable layer and the absorbent core from occurring and can effectively prevent the constituent fibers of the absorbent core (when the absorbent core also contains SAP particles, both the constituent fibers and SAP particles of the absorbent core) from leaking through the liquid-permeable layer from the non-covered region resulting from the interfacial delamination between the liquid-permeable layer and the absorbent core, even if the strength of the absorbent core is decreased due to liquid absorption. The weight ratio of the thermoplastic resin fibers to the water absorbent fibers of 1/9 to 5/5 is a necessary condition for achieving a joining strength in dry state of 1.53 to 7.65 N/25 mm and a joining strength in wet state of 0.95 to 4.34 N/25 mm.

In a preferred embodiment (Embodiment 10) of the absorbent article according to Embodiment 9, the joint section is a compressed section which integrates the liquid-permeable layer with the absorbent body in the thickness direction.

In a preferred embodiment (Embodiment 11) of the absorbent article according to Embodiments 1 to 10, the constituent fibers of the absorbent core are adhered to each other. In the absorbent article according to Embodiment 11, the absorbent core maintains a sufficient strength before and after liquid absorption (i.e., not only in dry state but also in wet state), since an advanced network is formed among the fibers due to the adhesion of the constituent fibers of the absorbent core. The modes of adhesion include, for example, adhesion among thermoplastic resin fibers or between thermoplastic resin fibers and water absorbent fibers by heat fusion of the thermoplastic resin fibers, adhesion among thermoplastic resin fibers by hydrogen bonds, adhesion among water absorbent fibers, or adhesion between thermoplastic resin fibers and water absorbent fibers, etc. When the absorbent core comprises the other fibers, the thermoplastic resin fibers and/or water absorbent fibers may be adhered to the other fibers. Since hydrogen bonds are cleaved by the liquid absorbed by the absorbent core, hydrogen bonds do not inhibit the swelling of SAP particles contained in the absorbent core.

In a preferred embodiment (Embodiment 12) of the absorbent article according to Embodiments 1 to 11, through-holes penetrating through the liquid-permeable layer are formed at an opening ratio of 5 to 70%. If the opening ratio of the through-holes is less than 5%, it is not possible to achieve a sufficient improvement in the liquid-permeability of the liquid-permeable layer by the formation of the through-holes, whereas if the opening ratio of the through-holes is more than 70%, reversion of a liquid from the absorbent core to the liquid-permeable layer becomes remarkable. In addition, if through-holes are formed through the liquid-permeable layer, the leakage of the constituent fibers of the absorbent core (when the absorbent core also contains SAP particles, both the constituent fibers and SAP particles of the absorbent core) through the liquid-permeable layer from the non-covered region easily occurs when the strength of the absorbent core is decreased due to liquid absorption, and therefore the fiber leakage prevention effect of the absorbent article (when the absorbent core also contains SAP particles, both the fiber leakage prevention effect and SAP particle leakage prevention effect) according to Embodiments 1 to 11 is remarkable in Embodiment 12.

In a preferred embodiment (Embodiment 13) of the absorbent article according to Embodiments 1 to 12, through-holes penetrating through the liquid-permeable layer and the absorbent core are formed. The absorbent article according to Embodiment 13 has improved absorbing properties and storage properties to liquids having a high viscosity. In addition, if through-holes are formed through the liquid-permeable layer and the absorbent core, the leakage of the constituent fibers of the absorbent core (when the absorbent core also contains SAP particles, both the constituent fibers and SAP particles of the absorbent core) through the liquid-permeable layer from the non-covered region easily occurs when the strength of the absorbent core is decreased due to liquid absorption, and therefore the fiber leakage prevention effect of the absorbent article (when the absorbent core also contains SAP particles, both the fiber leakage prevention effect and SAP particle leakage prevention effect) according to Embodiments 1 to 12 is remarkable in Embodiment 13.

In a preferred embodiment (Embodiment 14) of the absorbent article according to Embodiments 1 to 13, the thermoplastic resin fibers are core-sheath type conjugate fibers comprising as a sheath component a modified polyolefin having a vinyl monomer graft-polymerized thereto, or a mixed polymer of the modified polyolefin and other resins, and as a core component a resin having a melting point higher than that of the modified polyolefin, wherein the vinyl monomer comprises a unsaturated carboxylic acid, a unsaturated carboxylic acid anhydride, or a mixture thereof.

In a preferred embodiment (Embodiment 15) of the absorbent article according to Embodiments 1 to 14, the unsaturated carboxylic acid, unsaturated carboxylic acid anhydride, or mixture thereof is maleic acid or a derivative thereof, maleic anhydride or a derivative thereof, or a mixture thereof.

In a preferred embodiment (Embodiment 16) of the absorbent article according to Embodiments 1 to 15, the thermoplastic resin fibers contained in the absorbent core are colored. In the absorbent article according to Embodiment 16, it is easy to visually confirm whether or not the water absorbent fibers and the thermoplastic resin fibers are uniformly dispersed. In addition, the color of the absorbed liquid can be masked. For example, the wearer can feel clean when it is preliminary colored in a bluish color if the liquid to be absorbed is urine or in a greenish color if the liquid to be absorbed is menstrual blood.

The type and application of the absorbent article of the present invention are not particularly limited. Absorbent articles include, for example, hygiene articles and sanitary articles including sanitary napkins, disposable diapers, panty liners, incontinence pads, sweat sheets, etc., that may be intended for human or for animals such as pet animals, other than human.

The liquid to be absorbed by the absorbent article is not particularly limited, and includes, for example, liquid excrements (e.g., menstrual blood, urine, vaginal discharge, etc.) discharged from the wearer, etc.

Hereinafter, embodiments of the absorbent article of the present invention will be explained with reference to the drawings, taking a sanitary napkin as an example.

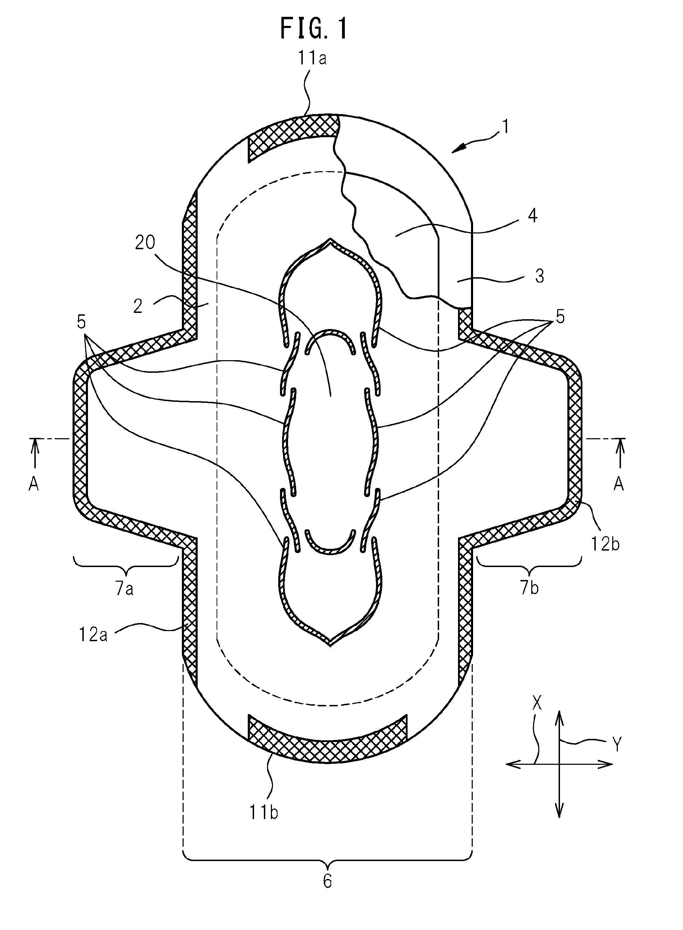

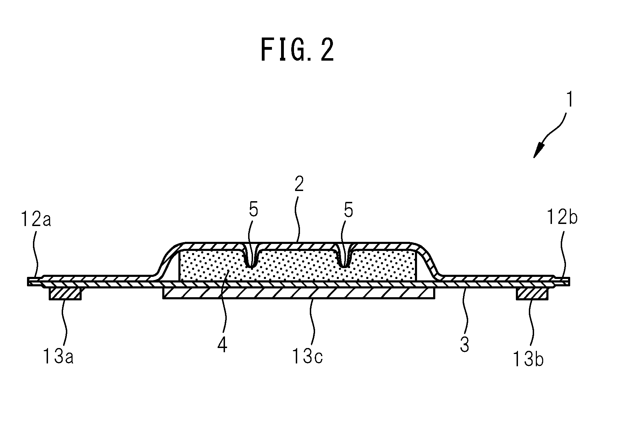

Sanitary napkin 1 according to one embodiment of the present invention comprises liquid-permeable top sheet 2, liquid-impermeable back sheet 3, absorbent core 4 disposed between liquid-permeable top sheet and liquid-impermeable back sheet 3, and compressed section 5 which integrates top sheet 2 with absorbent core 4 in the thickness direction, as shown in Figs. 1 and 2. In Fig. 1, the X-axis direction corresponds to the width direction of sanitary napkin 1, the Y-axis direction corresponds to the longitudinal direction of sanitary napkin 1, and the planar direction extending in the X-axis and Y-axis directions corresponds to the planar direction of sanitary napkin 1. This also applies to other figures.

Sanitary napkin 1 is worn for the purpose of absorbing liquid excrements (especially menstrual blood) discharged from the wearer. In this case, sanitary napkin is worn so that top sheet 2 is positioned at the skin side of the wearer and back sheet 3 is positioned at the clothing (underwear) side of the wearer. The liquid excrements discharged from the wearer reaches at absorbent core 4 through top sheet 2 and is absorbed by and retained in absorbent core 4. The leakage of the liquid absorbed by and retained in absorbent core 4 is prevented by back sheet 3.

As shown in Fig. 1, top sheet 2 and back sheet 3 are joined together at their edge sections in the longitudinal direction with seal sections 11a, 11b and thereby form body section 6, while the edge sections in the width direction are joined together with seal sections 12a, 12b and thereby form roughly rectangular wing sections 7a, 7b extending from body section 6 in the width direction.

The shape of the body section 6 may be appropriately modified within a range suitable for the female body, underwear, etc., and for example, a substantially rectangular shape, a substantially elliptical shape, a substantially hourglass shape, etc. The total dimension in the lengthwise direction of body section 6 is typically 100 to 500 mm, and preferably 150 to 350 mm, and the total dimension in the width direction of body section 6 is preferably 30 to 200 mm, and more preferably 40 to 180 mm.

The joint means for seal sections 11a, 11b, 12a, 12b, includes, for example, embossing, ultrasonic, hot-melt type adhesives, etc. To increase the joining strength, two or more bonding means may be combined (for example, bonding with a hot-melt adhesive, followed by embossing, etc.).

Embossing methods include, for example, a method in which top sheet 2 and back sheet 3 are passed together between a flat roll and an embossing roll having a convex part corresponding to the embossing pattern to be formed (a method so-called round sealing), etc. By this method, heating the embossing roll and/or flat roll softens each sheet, and accordingly the seal sections become more distinct. The embossing pattern includes, for example, lattice patterns, zigzag patterns, wavy patterns, etc. In order to make sanitary napkin 1 less bending at the borders of the seal sections, the emboss pattern is preferably intermittently and elongated.

The hot-melt adhesives include, for example, pressure-sensitive adhesives and heat-sensitive adhesives composed mainly of rubber-based materials such as styrene-ethylene-butadiene-styrene (SEBS), styrene-butadiene-styrene (SBS), styrene-isoprene-styrene (SIS), etc., or composed mainly of olefin-based materials such as linear low-density polyethylene, etc.; water-sensitive adhesives comprising a water-soluble polymer (such as polyvinyl alcohol, carboxylmethyl cellulose, gelatin, etc.) or water-swelling polymer (such as polyvinyl acetate, sodium polyacrylate, etc.), etc. Methods of applying an adhesive include, for example, spiral coating, coater coating, curtain coater coating, summitgun coating, etc.

As shown in Fig. 2, pressure-sensitive adhesive sections 13a, 13b are provided on the clothing side of back sheet 3 forming wing sections 7a, 7b, and a pressure-sensitive adhesive section 13c is attached to the clothing side of back sheet 3 forming body section 6. Pressure-sensitive adhesive section 13c is attached to the crotch section of underwear, while wing sections 7a, 7b are folded toward the external surface of the underwear and pressure-sensitive adhesive sections 13a, 13b are attached to the crotch section of the underwear, thereby stably anchor sanitary napkin 1 to the underwear.

The pressure-sensitive adhesive contained in pressure-sensitive adhesive section 13a, 13b, 13c includes, for example, styrene-based polymers such as styrene-ethylene-butylene-styrene block copolymer, styrene-butylene polymer, styrene-butylene-styrene block copolymer, styrene-isobutylene-styrene copolymer, etc.; tackifiers such as C5 petroleum resins, C9 petroleum resins, dicyclopentadiene-based petroleum resins, rosin-based petroleum resins, polyterpene resins, terpene phenol resins, etc.; monomeric plasticizers such as tricresyl phosphate, dibutyl phthalate and dioctyl phthalate, etc.; and polymeric plasticizers such as vinyl polymers, polyesters, etc.

Top sheet 2 is a sheet capable of transmitting the liquid excrements discharged from the wearer, and is an example of the liquid-permeable layer. One side of top sheet 2 is the side which will contact with the skin of the wearer.

Top sheet 2 is not particularly limited as long as the liquid excrements discharged from the wearer can permeate therethrough. Top sheet 2 includes, for example, nonwoven fabrics, woven fabrics, synthetic resin films having liquid-permeable pores formed therein, sheets in the form of net having a mesh, etc., and among these, nonwoven fabrics are preferred.

The fibers which constitute the nonwoven fabric include, for example, natural fibers (for example, wool, cotton, etc.), regenerated fibers (for example, rayon, acetate, etc.), inorganic fibers (for example, glass fibers, carbon fibers, etc.), synthetic resin fibers (for example, polyolefins such as polyethylene, polypropylene, polybutylene, ethylene-vinyl acetate copolymer, ethylene-ethyl acrylate copolymer, ethylene-acrylic acid copolymer, ionomer resins, etc.; polyesters such as polyethylene terephthalate, polybutylene terephthalate, polytrimethylene terephthalate, polylactic acid, etc.; and polyamides such as nylon, etc.). The nonwoven fabric may contain therein conjugate fibers such as core-sheath type fibers, side-by-side-type fibers, island/sea-type fibers, etc.; hollow-type fibers; profiled fibers such as flat fibers, Y-shaped fibers, C-shaped fibers, etc.; latent crimping or actually crimped three-dimensionally crimp fibers; splittable fibers capable of being split by a physical load such as water stream, heat, embossing, etc.

Methods for producing a non-woven fabric include, for example, a method in which a web (fleece) is formed and the fibers are physically or chemically bonded to each other. Methods for forming a web include, for example, spun bond methods, dry methods (carding methods, spunbond methods, meltblown methods, air-laid methods, etc.), wet methods, etc., and the bonding methods include, for example, thermal bonding methods, chemical bonding methods, needle punching methods, stitch bonding methods, spunlace methods, etc. Other than the nonwoven fabrics produced in such manners, a spunlace formed into a sheet form by hydroentangling method may be used as top sheet 2. In addition, a nonwoven fabric having irregularities on the skin-side surface (for example, a nonwoven fabric having on the upperlayer side irregularities formed by contracting the side of the underlayer containing heat-shrinkable fibers, a non-woven fabric having irregularities formed by applying air during web forming, etc.) may be used as top sheet 2. By forming irregularities in this manner on the skin side surface, it is possible to reduce the contact area between top sheet 2 and the skin.

Top sheet 2 preferably has through-holes penetrating through top sheet 2. If top sheet 2 has through-holes (for example, a perforated film, a perforated nonwoven fabric), the opening ratio (a proportion of the total surface of through-holes to the surface are of top sheet 2) of the through-holes is preferably 5 to 70 %, and more preferably 10 to 40%. If the opening ratio of the through-holes is less than 5%, it is not possible to achieve a sufficient improvement in the liquid-permeability of top sheet 2, whereas if the opening ratio of the through-holes is more than 70%, reversion of a liquid from absorbent core 4 to top sheet 2 becomes remarkable. The diameter of the through-holes is preferably 0.01 to 5 mm, and more preferably 0.5 to 3 mm, and the spacing between the through-holes is preferably 0.02 to 20 mm, and more preferably 1 to 10 mm.

If through-holes are formed through top sheet 2, the leakage of the constituent fibers of absorbent core 4 (if absorbent core 4 also contains SAP particles, both the constituent fibers and SAP particles of absorbent core 4) through top sheet 2 from the non-covered region easily occurs when the strength of absorbent core 4 is decreased due to absorption of a liquid excrement, and therefore the fiber leakage prevention effect of sanitary napkin 1 (if absorbent core 4 also contains SAP particles, the fiber leakage prevention effect and SAP particle leakage prevention effect) is remarkable when through-holes are formed through top sheet 2.

The thickness, basis weight, density, etc., of top sheet 2 can be appropriately adjusted within a range so that the liquid excrements discharged from the wearer can permeate therethrough. When a nonwoven fabric is used as top sheet 2, the fineness, fiber length and density of the fibers constituting the nonwoven fabric, the basis weight and thickness of the nonwoven fabric, etc., can be appropriately adjusted in view of the permeability to liquid excrements, skin touch, etc.

In view of increasing the concealing property of top sheet 2, an inorganic filler such as titanium oxide, barium sulfate, calcium carbonate, etc., may be added to the nonwoven fabric used as top sheet 2. When the fibers of the nonwoven fabric are core-sheath type conjugate fibers, an inorganic filler may be contained only in the core or may be contained only in the sheath.

Back sheet 3 is a sheet through which the liquid excrements discharged from the wearer cannot penetrate, and is an example of a liquid-impermeable layer. One side of the back seat is in contact with the clothing (underwear) of the wearer. Back sheet 3 preferably has a moisture permeability as well as liquid impermeability, in order to reduce stuffy feeling during wearing.

Back sheet 3 is not particularly limited as long as it cannot transmit the liquid excrements discharged from the wearer. Back sheet 3 includes, for example, waterproof-treated nonwoven fabrics, films of synthetic resins (such as polyethylene, polypropylene, polyethylene terephthalate, etc.), composite sheets comprising a nonwoven fabric and a synthetic resin film (for example, composite films having an air-permeable synthetic resin film joined to a nonwoven fabric such as spunbond nonwoven fabrics, spunlace nonwoven fabrics, etc.), and SMS nonwoven fabrics comprising a pair of high-strength spunbond nonwoven fabrics and a highly water-resistant meltblown nonwoven fabric placed therebetween.

An adhesive (for example, a hot-melt adhesive) is applied to the interface between top sheet 2 and absorbent core 4 and the interface between back sheet 3 and absorbent core 4, and top sheet 2 is joined to one side of absorbent core 4 and back sheet 3 is joined to the other side of absorbent core 4. In view of the liquid permeability from top sheet 2 to absorbent core 4, the adhesive is not applied to the entire interface between top sheet 2 and absorbent 4, but is applied in a pattern, such as dots, spiral, stripes, etc. Adhesive includes, for example, pressure-sensitive adhesives or heat-sensitive adhesives composed mainly of a rubber-based compound such as styrene-ethylene-butadiene-styrene (SEBS), styrene-butadiene-styrene (SBS), styrene-isoprene-styrene (SIS), etc., or composed mainly of an olefin-based compound such as linear low-density polyethylene, etc.; water-sensitive adhesives comprising water-soluble polymers (such as polyvinyl alcohol, carboxylmethyl cellulose, gelatin, etc.) or water-swelling polymers (such as polyvinyl acetate and sodium polyacrylate), etc. The application method for the adhesive includes, for example, spiral coating application, coater application, curtain coater application and summit-gun coating. Coating amount of the adhesive (basis weight) is typically 0.5 to 20 $g/m^2$, and preferably 2 to 10 $g/m^2$.

Absorbent core 4 comprises as constituent fibers cellulose-based water-absorbent fibers (hereinafter also abbreviated as "water absorbent fibers") and thermoplastic resin fibers (hereinafter also abbreviated as "thermoplastic resin fibers") comprising as a monomer component an unsaturated carboxylic acid, a unsaturated carboxylic acid anhydride, or a mixture thereof. The water absorbent fibers are responsible mainly for the liquid absorbing and retaining properties of absorbent core 4, and the thermoplastic resin fibers are responsible mainly for the strength (particularly the wet strength after liquid absorption) of absorbent core 4.

In a preferred embodiment, absorbent core 4 comprises, in addition to the cellulose-based water-absorbent fibers and thermoplastic resin fibers, superabsorbent resin (Superabsorbent Polymer: SAP) particles. SAP particles are responsible mainly for the liquid absorbing and retaining properties of absorbent core 4. The absorbing and retaining properties of absorbent core 4 are improved by the inclusion of SAP particles in absorbent core 4.

The water absorbent fibers and thermoplastic fibers (in a preferred embodiment, water absorbent fibers, thermoplastic fibers and SAP particles) are contained in a mixed state in absorbent core 4. The fibers are adhered to each other at their intersection points (for example, intersection points between thermoplastic resin fibers, intersection points between the thermoplastic resin fibers and water absorbent fibers) by heat fusion of the thermoplastic resin fiber. In addition, the fibers are mechanically entangled and are adhered to each other by hydrogen bonds formed between thermoplastic resin fibers, between water absorbent fibers, or between thermoplastic resin fibers and water absorbent fibers. If absorbent core 4 contains the other fibers, the thermoplastic resin fibers and/or water absorbent fibers may be adhered to the other fibers. If absorbent core 4 contains SAP particles, SAP particles may be adhered to the fibers by heat fusion of the thermoplastic resin fibers.

Since adhesion between the fibers contained in absorbent core 4 forms an advanced network between fibers, absorbent core 4 maintains a sufficient strength before and after liquid absorption (i.e., not only in dry state but also in wet state). Therefore, the collapse of absorbent core 4 as well as the leakage of the constituent fibers of absorbent core 4 through top sheet 2 from the non-covered region, resulting from the collapse of absorbent core 4, can be effectively prevented, even if the strength of absorbent core 4 is decreased due to liquid absorption. In addition, if absorbent core 4 contains SAP particles, the SAP particles are retained in the advanced network formed between the fibers, and thereby the leakage of the SAP particles from absorbent core 4 can be effectively prevented.

The heat fusion is carried out by, for example, heating the mixed material comprising the water absorbent fibers and the thermoplastic resin fibers (in a preferred embodiment, the water absorbent fibers, the thermoplastic fibers and the SAP particles) at a temperature which is equal to or higher than the melting point of the thermoplastic resin fibers. The heating temperature can be adjusted appropriately in accordance with the type of the thermoplastic resin fibers. The temperature which is equal to or higher than the melting point of the thermoplastic resin fibers may be a temperature at which a portion of the thermoplastic resin fibers will melt, and for example, when the thermoplastic resin fibers are core-sheath type conjugate fibers, the temperature may be equal to or higher than the temperature at which the sheath component will melt.

Absorbent core 4 has a non-covered region where the constituent fibers thereof are exposed to the surface so that the constituent fibers directly contact with top sheet 2. In this embodiment, absorbent core 4 is not covered with a core wrap, and therefore the constituent fibers of absorbent core 4 are exposed on the entire surface of absorbent core 4, and the proportion of the non-covered region to the interface between top sheet 2 and absorbent core 4 is 100%.

As long as absorbent core 4 has a non-covered region, absorbent core 4 may be covered with a core wrap. The core wrap is not particularly limited as long as it has liquid permeability and absorbent retention properties. The core wrap includes, for example, nonwoven fabrics, woven fabrics, synthetic resin films having liquid-permeable pores formed therein, sheets in the form of net having a mesh, etc.

For example, the surface of absorbent core 4 except for the interface between top sheet 2 and absorbent core 4 may be covered with a core wrap. In addition, the surface of absorbent core 4 may be covered with a core wrap at the interface between top sheet 2 and absorbent core 4. However, the higher the percentage of non-covered region to the interface between top sheet 2 and absorbent core 4, the greater the possibility to leak the constituent fibers of absorbent core 4 (if absorbent core 4 also contains SAP particles, the constituent fibers and SAP particles of absorbent core 4)

through top sheet 2 from the non-covered region. Thus, the fiber leakage prevention effect of sanitary napkin 1 (if absorbent core 4 also contains SAP particles, the fiber leakage prevention effect and SAP particle leakage prevention effect) is more remarkable with the increase in the proportion of the non-covered region to the interface between top sheet 2 and absorbent core 4. From such a view point, the proportion of the non-covered region to the interface between top sheet 2 and absorbent core 4 is preferably 10% or more, and more preferably 30% or more. The upper limit is 100%.

The proportion of the non-covered region to the interface between top sheet 2 and absorbent core 4 is calculated assuming that the region (absorbent core disposition region) where absorbent core 4 overlaps with top sheet 2 when absorbent core 4 is projected to top sheet 2 as being the interface between top sheet 2 and absorbent core 4, and assuming that a region of the absorbent core disposition region where a core wrap is not provided as being the non-covered region. Accordingly, the region where compressed section 5 has been formed and the region to which an adhesive has been applied between top sheet 2 and absorbent core 4 are included in the interface between top sheet 2 and absorbent core 4.

Sanitary napkin 1 may comprise as a liquid-permeable layer a second sheet disposed between top sheet 2 and absorber 4, in addition to top sheet 2. A sheet such as nonwoven fabrics, etc., illustrated for top sheet 2, can be appropriately selected and used as a second sheet. If a second sheet is disposed between top sheet 2 and absorbent 4, the fiber leakage prevention effect of sanitary napkin 1 (if absorbent core 4 also contains SAP particles, the fiber leakage prevention effect and SAP particle leakage prevention effect) is more remarkable with the increase in the proportion of the non-covered region to the interface between the second sheet and absorbent core 4. Therefore, the proportion of the non-covered region to the interface between the second sheet and absorbent core 4 is preferably 10% or more, and more preferably 30% or more, as in the above case. The upper limit is 100%.

The weight ratio of the thermoplastic resin fibers to the water absorbent fibers (thermoplastic resin fibers/water absorbent fibers) in absorbent core 4 is 1/9 or more. The lower limit of 1/9 has been determined in view of the strength (particularly the wet strength after liquid absorption) of absorbent core 4, and if the weight ratio of the thermoplastic resin fibers to the water absorbent fibers is 1/9 or more, absorbent core 4 maintains a sufficient strength before and after liquid absorption (i.e., not only in dry state but also in wet state).

The greater the weight ratio of the thermoplastic resin fibers to the water absorbent fibers (thermoplastic fiber/absorbent fiber) in absorbent core 4, the greater the strength of absorbent core 4. The strength of absorbent core 4 increases with the increase in the weight ratio of the thermoplastic resin fibers to the water absorbent fibers, for example, from 1/9 to 1.5/8.5, 2/8, 2.5/7.5, 3/7, 3.5/6.5, 4/6, and 4.5/5.5. Therefore, the weight ratios of 1/9, 1.5/8.5, 2/8, 2.5/7.5, 3/7, 3.5/6.5, 4/6, and 4.5/5.5 may have significances as a lower limit of the weight ratio of the thermoplastic resin fibers to the water absorbent fibers, in view of increasing the strength of absorbent core 4.

Absorbent core 4 has a wet fiber falling-off rate of 13.3% or less. Accordingly, sanitary napkin 1 can effectively prevent absorbent core 4 from collapsing and can effectively prevent the constituent fibers of absorbent core 4 from leaking through top sheet 2 from the non-covered region resulting from the collapsing of absorbent core 4, even if the strength of absorbent core 4 is decreased due to absorption of a liquid excrement. Therefore, sanitary napkin 1 is less likely to give uncomfortable feeling to the wearer due to fiber leakage.

Absorbent core 4 preferably has a fiber falling-off rate in dry state of 2.8% or less, and absorbent core 4 has a SAP particle falling-off rate in dry state of 14.3% or less, more preferably 10% or less, and even more preferably 5% or less. Accordingly, in sanitary napkin 1, the leakage of the fibers and SAP particles from absorbent core 4 can be effectively prevented, even before absorption of a liquid excrement.

The weight ratio of the thermoplastic resin fibers to the water absorbent fibers of 1/9 has a critical significance in that the fiber falling-off rates in dry and wet states (particularly fiber falling-off rate in wet state) of absorbent core 4 when the weight ratio of the thermoplastic fibers to the water absorbent fibers is less than 1/9 differs significantly from the fiber falling-off rates in wet and dry states when the weight ratio is equal to or more than 1/9 (see Experiment Example I-1). Therefore, in view of decreasing the fiber falling-off rates in dry and wet states of absorbent core 4, it is advantageous to adjust the weight ratio of the thermoplastic resin fibers to the water absorbent fibers in absorbent core 4 to 1/9 or more, and thereby a fiber falling-off rate in dry state of 2.8% or less for absorbent core 4 and a fiber falling-off rate in wet state of 13.3% or less for absorbent core 4 can be achieved.

The greater the weight ratio of the thermoplastic resin fibers to the water absorbent fibers (thermoplastic resin fibers/water absorbent fibers) in absorbent core 4, the lower the fiber falling-off rates in dry and wet states and SAP particle falling-off rate in dry state of absorbent core 4. For example, the fiber falling-off rates in dry and wet states and SAP particle falling-off rate of absorbent core 4 decrease with the increase in the weight ratio of the thermoplastic resin fibers to the water absorbent fibers, for example, from 1/9 to 1.5/8.5, 2/8, 2.5/7.5, 3/7, 3.5/6.5, 4/6, and 4.5/5.5. Therefore, the weight ratios of 1.5/8.5, 2/8, 2.5/7.5, 3/7, 3.5/6.5, 4/6, and 4.5/5.5 may have significances as a lower limit of the weight ratio of the thermoplastic resin fibers to the water absorbent fibers, in view of decreasing the fiber falling-off rates in dry and wet states and SAP particle falling-off rate in dry state of absorbent core 4. For example, the weight ratio of the thermoplastic resin fibers to the water absorbent fibers can be adjusted to 2/8 or more, and thereby it is possible to achieve a fiber falling-off rate in dry state of 1.5% or less for absorbent core 4 and a fiber falling-off rate in wet state of 12.8% or less for absorbent core 4, as well as a SAP particle falling-off rate in dry state of 10.3% or less for absorbent core 4.

The upper limit of the weight ratio of the thermoplastic resin fibers to the water absorbent fibers (thermoplastic resin fibers/water absorbent fibers) in absorbent core 4 is preferably 5/5. The upper limit of 5/5 has been determined in view of the liquid absorbing properties of absorbent core 4, and if the weight ratio of the thermoplastic resin fibers to the water absorbent fibers is 5/5 or less, absorbent core 4 also has a sufficient liquid absorbing properties. This leads to the improvement in the liquid permeability and diffusibility of absorbent core 4, and thereby, when absorbent core 4 contains SAP particles, the liquid absorbing and retaining properties of the SAP particles can be effectively exerted.

The smaller the weight ratio of the thermoplastic resin fibers to the water absorbent fibers (thermoplastic fibers/absorbent fibers) in absorbent core 4, the weaker the effects of the hydrophobicity of the thermoplastic resin fibers and thereby increasing the liquid absorbing properties of absorbent core 4. The liquid absorbing properties of absorbent core 4 increase with the decrease in the weight ratio of the thermoplastic resin fibers to the water absorbent fibers, for example, from 5/5 to 4.5/5.5, 4/6, 3.5/6.5, 3/7, 2.5/7.5, 2/8, and 1.5/8.5. Therefore, the weight ratios of 5/5, 4.5/5.5, 4/6, 3.5/6.5, 3/7, 2.5/7.5, 2/8, and 1.5/8.5 may have significances as an upper limit of the weight ratio of the thermoplastic resin fibers to the water absorbent fibers, in view of increasing the liquid absorbing properties of absorbent core 4.

The weight ratio of the thermoplastic resin fibers to the water absorbent fibers in absorbent core 4 (thermoplastic resin fibers/water absorbent fibers) is preferably 1/9 to 5/5, and more preferably 2/8 to 4/6, in view of the strength (particularly strength in wet state after liquid absorption) and liquid absorbing properties of absorbent core 4. This leads absorbent core 4 to have a sufficient strength (particularly strength in wet state after liquid absorption) and a sufficient liquid absorbing properties.

If the weight ratio of the thermoplastic resin fibers to the water absorbent fibers in absorbent core 4 (thermoplastic resin fibers/water absorbent fibers) is 1/9 to 5/5, it is possible to achieve a fiber falling-off rate in dry state of 0.9 to 2.8% for absorbent core 4 and a fiber falling-off rate in wet state of 1.0 to 13.3% for absorbent core 4, and it is possible to achieve a fiber falling-off rate in dry state of 3.7 to 14.3% for absorbent core 4.

Desired fiber falling-off rates in dry and wet states and SAP particle falling-off rate in dry state can be achieved by adjusting the weight ratio of the thermoplastic resin fibers to the water absorbent fibers in absorbent core 4 and then appropriately adjusting the preparation conditions (for example, heating conditions during heat fusing) for absorbent core 4. For example, desired fiber falling-off rates in dry and wet states can be achieved by blowing a hot air of 130 to 220°C and preferably 140 to 180°C to a mixed material comprising water absorbent fibers and thermoplastic resin fibers at an air flow of 2.5 to 30 m/sec., and preferably 5 to 20 m/sec., for 0.5 to 60 seconds, and preferably for 5 to 30 seconds. Blowing of a hot air can be carried out by, for example, an air-through method. Here, blowing of a hot air is an example of heat treatment. The heat treatment is not particularly limited as long as heating at a temperature which is equal to or higher than the melting point of the thermoplastic resin fibers is possible. In addition to a hot air, the heat treatment can be carried out using a heating medium such as microwave, steam, infrared radiation, etc.

The fiber falling-off rate in dry state (%) of absorbent core 4 is measured for in the following manner.

An empty vessel is charged with 100 mm × 100 mm pre-test sample pieces and is shaken with a shaker (for example, SHKV-200 manufactured by IWAKI Co.) at a shaking rate of 300 rpm for 1 hour, and a sample piece which maintains a sheet form after shaking is removed from the vessel and is marked as a sample piece after testing. Then, a fiber falling-off rate (%) (the weight of the fallen fibers/the weight of the pre-test sample piece × 100) is calculated based on the weight of the fallen fibers (the weight of the pre-test sample piece minus the weight of the post-test sample piece).

The fiber falling-off rate in wet state (%) of absorbent core 4 is measured in the following manner.

A vessel containing distilled water is charged with 100 mm × 100 mm pre-test sample pieces and is shaken with a shaker (for example, SHKV-200 manufactured by IWAKI Co., Ltd.) at a shaking rate of 250 rpm for 30 seconds, and a sample piece which maintains a sheet form after shaking is removed from the vessel, is dried sufficiently, and is marked as a post-test sample piece. Then, a fiber falling-off rate (%) (the weight of the fallen fibers/the weight of the pre-test sample piece × 100) is calculated based on the weight of the fallen fibers (the weight of the pre-test sample piece minus the weight of the post-test sample piece). The amount of the distilled water in the vessel is adjusted to an amount (for example, 1000 mL) such that the sample piece is sufficiently immersed therein. Commercial dryers, for example, an air-blowing, constant-temperature thermostat (DNE-910 manufactured by Yamato Scientific Co., Ltd.) can be used in drying, and the drying temperature may be set at, for example, 80°C and the drying time may be set at, for example, 12 hours or more.

The SAP particle falling-off rate in dry state (%) of absorbent core 4 is measured in the following manner.

An empty vessel is charged with 100 mm × 100 mm pre-test sample pieces cut out from absorbent core 4 and is shaken with a shaker (for example, SHKV-200 manufactured by IWAKI Co., Ltd.) at a shaking rate of 300 rpm for 10 minutes, and a sample piece which maintains a sheet form after shaking is removed from the vessel and is marked as a post-test sample piece. Then, a fiber falling-off rate (%) (the weight of the fallen fibers/the weight of the pre-test sample piece × 100) is calculated based on the weight of the fallen fibers (the weight of the pre-test sample piece minus the weight of the post-test sample piece).

Absorbent core 4 has a fiber density of preferably 0.06 to 0.14 g/cm$^3$, more preferably 0.07 to 0.12 g/cm$^3$, and even more preferably 0.08 to 0.1 g/cm$^3$. If the fiber density of absorbent core 4 is 0.06 to 0.14 g/cm$^3$ when the weight ratio of the thermoplastic resin fibers to the water absorbent fibers in absorbent core 4 is 1/9 to 5/5, sufficient liquid absorbing properties can be imparted to absorbent core 4.

If absorbent core 4 comprises the fibers and SAP particles, the fiber density of absorbent core 4 is calculated on the basis of the following equation.

$$\mathtt{FD~(g/cm^3)~=~FB~(g/m^2)~/~T~(mm)~\times~10^{-3}}$$

wherein FD, FB and T are the fiber density, fiber basis weight and thickness of absorbent core 4, respectively.

The fiber basis weight (g/m$^2$) of absorbent core 4 is calculated on the basis of the following equation.

$$\mathtt{FB~(g/m^2)~=~B~(g/m^2)~\times~FR}$$

wherein FB is as defined above, B represents the basis weight of absorbent core 4, and FR represents the fiber mass ratio in absorbent core 4.

The measurement on the basis weight (g/m$^2$) of absorbent core 4 is carried out as follows.

Three 100mm × 100 mm sample pieces are cut out from absorbent core 4 and the weight is measured for each sample piece under standard condition (temperature 23 ± 2°C, relative humidity 50 ± 5%) with a direct reading balance (for example, Electronic Balance HF-300 manufactured by Kensei Co., Ltd.), and the weight per unit surface area (g/m$^2$) of absorbent core 4 calculated from the average value of the three measurement values is determined as the basis weight of absorbent core 4.

Regarding the measurement on the basis weight of absorbent core 4, the measurement conditions described in ISO 9073-1 or JIS L 1913 6.2 are used for the measurement conditions other than those specified above.

The measurement on the fiber weight ratio in absorbent core 4 is carried out as follows.

One gram each of a SAP particle sample and fiber sample was collected from absorbent core 4. At that time, it is preferable to loosen absorbent core 4 and to collect the SAP particles and fibers of interest using a magnifying glass. Next, the sample of the collected SAP particles is placed in a 250 mesh nylon bag (in dry state) to measure the water capacity thereof. In the same manner, the sample of the collected fibers is placed in a 250 mesh nylon bag (in dry state) to measure the water capacity thereof. The measurement on the water capacity is carried as follows.

The sample filled nylon bags are thoroughly immersed in a 500 ml of 0.9% physiological saline for 30 minutes and subsequently are dehydrated for 2 minutes at 150 G with a centrifugal separator, and then the weights of the dehydrated samples are measured.

Meanwhile, the 250 mesh nylon bags alone (in dry state) before placing the samples therein are thoroughly immersed in a 500 ml of 0.9% physiological saline for 30 minutes and subsequently are dehydrated for 2 minutes at 150 G with a centrifugal separator, and then the weights of the samples of the dehydrated nylon mesh bag alone are measured.

The following formula is used to calculate the water capacity (g/g) of the SAP particle sample and the water capacity (g/g) of the fiber sample.

Water capacity (g/g) of a sample = (the weight of the sample after dehydration - the weight of the sample before immersion - the weight of the nylon mesh bag alone after dehydration)/(the weight of the sample before immersion)

In this example, the weight of the sample before immersion was 1 g for both the SAP particle sample and fiber sample.

A 50 mm×50 mm core sample is cut out from absorbent core 4, and the weight W (g) of the core sample is measured. The size of the core sample can be adjusted in accordance with the size of the absorbent core, and is preferably as large as possible. Next, the core sample is placed in a 250 mesh nylon mesh bag to measure the water capacity thereof. The measurement on the water capacity is carried as follows. The nylon bag is thoroughly immersed in a 500 ml of 0.9% physiological saline for 30 minutes and subsequently is dehydrated for 10 minutes at 150 G with a centrifugal separator, and then the weight of the dehydrated core sample is measured and the water capacity (g/g) of the core sample is calculated based on the above formula.

When the weight of the SAP particles in the core sample is represented as S (g), and the weight of the fibers in the core sample is represented as P (g) (W = S + P), the following equation is established.

$$\text{Water capacity of the core sample (g/g)} \times \text{W (g)} =$$

$$\text{water capacity of the SAP particle sample (g/g)} \times \text{(W-P)}$$

$$\text{(g) + water capacity of the fiber sample (g/g)} \times \text{P (g).}$$

On the basis of this equation, the fiber weight ratio (P/W) in absorbent core 4 is calculated.

The measurement on the thickness (mm) of absorbent core 4 is carried out as follows.

The thicknesses of five different points of absorbent core 4 under standard condition (temperature 23 ± 2°C, relative humidity 50 ± 5%) are measured with a thickness gauge (for example, FS-60DS manufactured by Daiei Kagaku Seiki Manufacturing Co., Ltd., measurement surface 44 mm (diameter), measurement load 3 g/cm$^2$) by loading at a constant pressure of 3 g/cm$^2$ and measuring the thickness after 10 seconds pressure loading at each point, and an average value of the five measurement values is determined as the thickness of absorbent core 4.

The fiber density of absorbent core 4 can be adjusted to a desired range by densifying the mixed material comprising the water absorbent fibers and thermoplastic resin fibers. To maintain the fiber density of absorbent core 4 in a certain range, it is necessary to maintain the bulkiness of absorbent core 4 in a predetermined range by suppressing the elastic recovery of the fibers. In this regard, hydrogen bonds (for example, hydrogen bonds formed between water absorbent fibers, between the thermoplastic resin fibers, between water absorbent fibers and thermoplastic resin fibers, etc.) contribute to the maintenance of the bulkiness of absorbent core 4. Hydrogen bonds are formed, for example, between the oxygen atoms of the thermoplastic resin fibers (such as the oxygen atoms of carboxyl groups, acyl groups, ether bonds, etc.) and hydrogen atoms of cellulose (for example, hydrogen atoms of hydroxyl groups). In addition, since hydrogen bonds are cleaved by the liquid absorbed by absorbent core 4, hydrogen bonds do not inhibit the swelling of the absorbent materials (a water absorbent material which is an essential component and a superabsorbent material which is an optional component) included in absorbent core 4.

The maximum tensile strength in dry state of absorbent core 4 (the maximum tensile strength at a fiber basis weight of 200 g/m$^2$) is preferably 3 to 36 N/25 mm, and more preferably from 8 to 20 N/25 mm, and the maximum tensile strength in wet state of absorbent core 4 (the maximum tensile strength at a fiber basis weight of 200 g/m$^2$) is preferably 2 to 32 N/25 mm, and more preferably 5 to 15 N/25 mm. In addition, the maximum tensile strength in dry state of absorbent core 4 (the maximum tensile strength at a fiber basis weight of 100 g/m$^2$ and a SAP basis weight of 100 g/m$^2$) is preferably 1 to 18 N/25 mm, and more preferably from 2 to 10 N/25 mm, and the maximum tensile strength in wet state of absorbent core 4 (the maximum tensile strength at a fiber basis weight of 100 g/m$^2$ and a SAP basis weight of 100 g/m$^2$) is preferably 0.9 to 16 N/25 mm, and more preferably 2 to 10 N/25 mm. Thus, absorbent article 4 maintains a sufficient strength before and after liquid absorption (i.e., not only in dry state but also in wet state). The weight ratio of the thermoplastic resin fibers to the water absorbent fibers of 1/9 or more is a necessary condition for achieving such strength for absorbent core 4. Herein, "N/25 mm" means the maximum tensile strength (N) per 25 mm width in the planar direction of absorbent core 4, and the planar direction of absorbent core 4 incudes, for example, the conveyance direction (MD direction) of absorbent core 4 during manufacturing, the direction (CD direction) perpendicular to the MD direction, etc., and the planar direction is preferably the MD direction.

The maximum tensile strength in dry state of absorbent core 4 can be measured in the following manner.

A sample piece (150 mm length × 25 mm width) under standard condition (under an atmosphere of a temperature of 20°C and a humidity of 60%) is attached to a tensile tester (AG-1kNI manufactured by Shimadzu Corporation) at a clamping interval of 100 mm and is loaded at a tensile rate of 100 mm/min. until the sample piece is ruptured (the maximum load) to determine the maximum tensile strength (N/25 mm). Herein, "N/25 mm" means the maximum tensile strength (N) per 25 mm width in the lengthwise direction of the sample piece.

The maximum tensile strength in wet state of absorbent core 4 can be measured in the following manner.

After immersing a sample piece (150 mm length × 25 mm width) in ion-exchanged water until it is settled down by its own weight or after sinking the sample piece in the water for 1 hour, the maximum tensile strength (N/25 mm) is measured in the same manner as described for the maximum tensile strength in dry state. Herein, "N/25 mm" means the maximum tensile strength (N) per 25 mm width in the lengthwise direction of the sample piece.

Regarding the measurements of the maximum strengths in dry and wet states, the measurement conditions described in ISO 9073-3 or JIS L 1913 6.3 are used for the measurement conditions other than those specified above.

The difference between the maximum tensile strength in dry state and the maximum tensile strength in wet state of absorbent core 4 (the maximum tensile strength in dry state minus the maximum tensile strength in wet state) is preferably 1 to 5 N/25 mm, and more preferably 2 to 4 N/25 mm is. Thus, absorbent core 4 can maintain a sufficient strength before and after liquid absorption (i.e., not only in dry state but also in wet state). The weight ratio of the thermoplastic resin fibers to the water absorbent fibers of 1/9 or more is a necessary condition for achieving such strength for absorbent core 4. Since the hydrogen bonds formed in dry state will be cleaved in wet state, the difference between the maximum tensile strength in dry state and the maximum tensile strength in wet state is an index of the amount of hydrogen bonds.

The cellulose-based water absorbent fibers contained in absorbent core 4 include, for example, wood pulps (for example, mechanical pulps such as ground pulp, refiner ground pulp, thermomechanical pulp, chemithermomechanical pulp, etc.; chemical pulps such as kraft pulp, sulfide pulp, alkaline pulp, etc.; semichemical pulp, etc.) obtained from softwood or hardwood as a raw material; mercerized pulp obtained by subjecting a wood pulp to a chemical treatment or crosslinked pulp; non-wood pulps such as bagasse, kenaf, bamboo, hemp, cotton (for example, cotton linters), and the like; regenerated fibers such as rayon fiber, etc.

The thermoplastic fibers contained in absorbent core 4 is not particularly limited as long as it comprises as a monomer component a unsaturated carboxylic acid, unsaturated carboxylic acid anhydride or a mixture thereof, and may be appropriately selected in view of strength, hydrogen bonding properties, heat fusing properties, etc.

The thermoplastic resin fibers contained in absorbent core 4 include, for example, core-sheath type conjugate fibers, etc., wherein the core-sheath type conjugate fibers comprise as a sheath component a modified polyolefin having a vinyl monomer graft-polymerized thereto, or a mixed polymer of the modified polyolefin and other resins, and as a core component a resin having a melting point higher than that of the modified polyolefin, wherein the vinyl monomer comprises a unsaturated carboxylic acid, a unsaturated carboxylic acid anhydride, or a mixture thereof.

The unsaturated carboxylic acids or unsaturated carboxylic acid anhydrides include, for example, vinyl monomers such as maleic acid or derivatives thereof, maleic anhydride or derivatives thereof, fumaric acid or derivatives thereof, unsaturated derivatives of malonic acid, unsaturated derivatives of succinic acid, etc., and other vinyl monomers include general purpose monomers having free-radical polymerization properties, for example, styrenes such as styrene, $\alpha$-methylstyrene, etc.; (meth)acrylate esters such as methyl (meth)acrylate, ethyl (meth)acrylate, 2-hydroxyethyl (meth)acrylate, dimethylaminoethyl (meth)acrylate, etc. The derivatives of maleic acid or derivatives of maleic anhydride include, for example, citraconic acid, citraconic anhydride, pyrocinconic acid, etc. The derivatives of fumaric acid or unsaturated derivatives of malonic acid include, for example, 3-butene-1,1-dicarboxylic acid, benzylidene malonic acid, isopropylidene malonic acid, etc. The unsaturated derivatives of succinic acid include, for example, itaconic acid, itaconic anhydride, etc.

The backbone polymers of the modified polyolefins includes, linear low density polyethylene (LLDPE), low density polyethylene (LDPE), medium density polyethylene (MDPE), high density polyethylene (HDPE), polypropylene, polybutylene, copolymers comprising mainly of these components (for example, ethylene-vinyl acetate copolymer (EVA), ethylene-ethyl acrylate copolymer (EEA), ethylene-acrylic acid copolymer (EAA), ionomer resins, etc.).

Graft polymerization of the vinyl monomer to the backbone polymer can be carried out by, for example, a method in which a polyolefin is mixed with a unsaturated carboxylic acid or unsaturated carboxylic acid anhydride along with a vinyl monomer to introduce a side chain comprising a random copolymer to the polyolefin with a radical initiator; a method in which different kinds of monomers are sequentially polymerized to introduce a side chain comprising a block copolymer to the polyolefin, etc.

The sheath component may be comprised of a modified polyolefin alone, or may be a mixed polymer of a modified polyolefin and other resins. Other resins are preferably polyolefins, and the same type of polyolefin as that of the backbone polymer is more preferable. For example, if the backbone polymer is a polyethylene, it is preferred that the other resin is also a polyethylene.

The resin used as the core component is not particularly limited as long as the resin has a melting point higher than that of the modified polyolefin and includes, for example, polyamides such as 6-Nylon, 6,6-Nylon, etc.; polyesters of a linear or branched chain polyhydroxy alkanoic acid having carbon atoms of up to 20, including polyethylene terephthalate (PET), poly(trimethylene terephthalate) (PTT), polybutylene terephthalate (PBT), polylactic acid, polyglycolic acid, etc., and copolymers comprising mainly of these polyesters, or copolymerized polyesters formed by copolymerizing as a main component an alkylene terephthalate with a minor amount of other components. PET is preferred from the viewpoint of high cushioning properties due to having elastic resilience, from the economic viewpoint of industrial availability at a low cost.

Spinning is possible if the conjugate ratio of the sheath component to the core component is in the range of from 10/90 to 90/10, and the conjugate ratio is preferably from 30/70 to 70/30. If the sheath component proportion is too low, heat fusibility is reduced, and if the sheath component proportion is too high, the spinnability is reduced.

Additives such as antioxidants, light stabilizers, UV absorbers, neutralizing agents, nucleating agents, epoxy stabilizers, lubricants, antimicrobial agents, flame retardants, antistatic agents, pigments, and plasticizers may be added to the thermoplastic resin fibers contained in absorbent core 4, if necessary. The thermoplastic resin fibers are preferably hydrophilized with a surface active agent, a hydrophilizing agent, etc.

Although the fiber length of the thermoplastic resin fibers contained in absorbent core 4 is not particularly limited, if it is mixed with a pulp in air-laid method, the fiber length is preferably 3 to 70 mm, and more preferably 5 to 20 mm. If the fiber length is less than this range, the number of the junctions with the water absorbent fibers is reduced, and therefore a sufficient strength cannot be imparted to absorbent core 4. On the other hand, if the fiber length exceeds the above range, the fibrillation properties are significantly decreased and thereby many fibers in unfibrillated state are generated, and therefore the texture unevenness is generated and the uniformity of absorbent core 4 is reduced. In addition, the fineness of the thermoplastic resin fibers is preferably 0.5 to 10 dtex, and more preferably 1.5 to 5 dtex. If the fineness is less than 0.5 dtex, the fineness is reduced, and if the fineness is more than 10 dtex, the number of fibers is reduced and thereby the strength is decreased.

The thermoplastic resin fibers contained in absorbent core 4 may be imparted with a three-dimensional crimped shape. Consequently, even if the fiber orientation is aligned to the planar direction, the buckling strength of the fiber exerts in the thickness direction, thereby making the fiber harder to crush even if an external force is applied thereto. The three-dimensional crimped shape includes, for example, a zigzag shape, and a Omega shape, a spiral shape, etc., and the method for imparting a three-dimensional crimped shape includes, for example, mechanical crimping, shaping by heat shrinking, etc. Mechanical crimping can be controlled by the peripheral speed difference in line speed, heat, pressurization, etc., with respect to continuous linear fibers after spinning, and the greater the number of crimps per unit length of the crimped fibers, the greater the buckling strength of the fibers under external pressure. The number of crimps is typically 10 to 35 per inch, and preferably 15 to 30 per inch. Shaping by heat shrinking can provide a three-dimensional crimping by using the difference in heat shrinking resulting from the melting temperature difference by, for example, heating fibers comprising two or more resins having different melting points. The cross-sectional shapes of the fibers include, for example, eccentric-type, side-by-side type of core-sheath type conjugate fibers. Such fibers have a heat shrinking rate of preferably 5 to 90%, and more preferably 10 to 80%.

In a preferred embodiment, absorbent core 4 comprises the cellulose-based water absorbent fibers and thermoplastic resin fibers, as well as superabsorbent resin (Superabsorbent Polymer: SAP) particles. The superabsorbent material which constitutes the SAP particles includes, for example, starch-based, cellulose-based, and synthetic polymer-based superabsorbent materials. The starch-based or cellulose-based superabsorbent material includes, for example, starchacrylic acid (salt) graft copolymers, saponified products of starch-acrylonitrile copolymers, crosslinked products of sodium carboxymethylcellulose, etc., and the synthetic polymer-based, superabsorbent material includes, for example, superabsorbent resins (Superabsorbent Polymer: SAP) of polyacrylic acid salt-based, polysulfonic acid salt-based, maleic anhydride salt-based, polyacrylamide-based, polyvinyl alcohol-based, polyethylene oxide-based, polyaspartic acid salt-based, polyglutamic acid-based, polyalginic acid salt-based, starch-based, cellulose-based types, etc., and of these, polyacrylic acid salt-based (particularly sodium polyacrylate-based) superabsorbent resins are preferred. The SAP particles may contain superabsorbent materials having the other shapes (e.g., fibrous, scaly, etc.).

The basis weight of the SAP particles contained in the absorbent core is typically 5 to 500 $g/m^2$, preferably 100 to 400 $g/m^2$, and more preferably 150 to 300 $g/m^2$. The basis weight ratio of the fibers to the SAP particles (the basis weight of the SAP particles/the basis weight of the fibers) in absorbent core 4 is typically 5/40 to 500/900, preferably 100/100 to 400/500, and more preferably 150/150 to 300/400. The particle size of the SAP particles is typically 50 to 1000 $\mu$m, preferably from 100 to 900 $\mu$m, and more preferably 300 to 700 $\mu$m. The measurement on the particle size of the SAP particles is carried in accordance with the sieving test method described in JIS R 6002:1998.

The thickness, fiber basis weight, etc., of absorbent core 4 can be adjusted appropriately in accordance with the properties (for example absorbing properties, strength, light weight properties, etc.) required for sanitary napkin 1. The thickness of absorbent core 4 is typically 0.1 to 15 mm, preferably from 1 to 10 mm, and more preferably 2 to 5 mm, and the fiber basis weight is typically 20 to 1000 $g/m^2$, preferably 40 to 900 $g/m^2$, and more preferably 100 to 400 $g/m^2$. If the fiber basis weight is less than 40 $g/m^2$, the fiber content of the thermoplastic resin fibers is insufficient, and therefore there is a possibility that the strength (particularly the strength in wet state after liquid absorption) of absorbent core 4 cannot be maintained, whereas if the fiber basis weight is more than 900 $g/m^2$, the fiber amount of the thermoplastic resin fibers is excessive, and therefore absorbent core 4 may have a too high rigidity. In addition, the thickness, fiber basis weight, etc., of absorbent core 4 may be constant throughout absorbent core 4 or may be partially varied.

Absorbent core 4 may be integrated with top sheet 2 by the through-holes penetrating through top sheet 2 and absorbent core 4. Consequently, the absorbing properties and storage properties to liquid having a high viscosity (for example, menstrual blood) are improved. In addition, if through-holes are formed through the liquid-permeable layer and the absorbent core, the leakage of the constituent fibers of absorbent core 4 (when absorbent core 4 also contains SAP particles, both the constituent fibers and SAP particles of absorbent core 4) through top sheet 2 from the non-covered region easily occurs when the strength of absorbent core 4 is decreased due to absorption of a liquid excrement, and therefore the fiber leakage prevention effect of sanitary napkin 1 (when absorbent core 4 also contains SAP particles, both the fiber leakage prevention effect and SAP particle leakage prevention effect) is remarkable. The opening ratio (a proportion of the total surface of through-holes to the surface are of top sheet 2) of the through-holes penetrating through top sheet 2 and absorbent core 4 is preferably 0.1 to 20 %, and more preferably 1 to 10%, the diameter of the through-holes is preferably 0.1 to 5 mm, and more preferably 0.5 to 3 mm, and the spacing between the through-holes is preferably 0.2 to 30 mm, and more preferably 5 to 20 mm.

The thermoplastic fibers contained in absorbent core 4 may be colored by a coloring matter, etc. This facilitates visual recognition as to whether the water absorbent fibers and thermoplastic resin fibers are uniformly dispersed or not. In addition, it is possible to mask the color of the absorbed liquid. For example, the wearer can feel clean if it is colored in a bluish color when the liquid to be absorbed is urine and if it is colored in a greenish color when the liquid to be absorbed is menstrual blood.

In order to impart absorbent core 4 with a desired function, silver, copper, zinc, silica, activated carbon, aluminosilicate compounds, zeolites, etc., may be added to absorbent core 4. Consequently, it is possible to impart functions such as deodorant, antibacterial, heat absorption effect, etc.

As shown in Fig. 1, compressed sections 5 are intermittently formed on the periphery or around excretory opening contact region 20 of the skin contact surface of top sheet 2. The formation pattern of compressed section 5 may be appropriately modified, and the formation pattern when viewed from top sheet 2 includes, for example, linear, curved, cyclic, dots, etc.

Excretory opening contact region 20 is a region with which the excretory opening (for example, labia minora, labia majora, etc.) of the wearer contacts when sanitary napkin 1 is worn by the wearer. Excretory opening contact region 20 is provided substantially in the center of the absorbent core disposition region. The position, surface area, etc., of excretory opening contact region 20 can be appropriately adjusted. Although excretory opening contact region 20 may be provided as a region which is substantially the same as the region with which excretory opening actually contacts or may be provided as a region which is larger than the region with which excretory opening actually contacts, it is preferable to provide the excretory opening contact region as a region which is larger than the region with which excretory opening actually contacts in view of preventing liquid excrements such as menstrual blood from leaking out to the outside. The length of the excretory opening contact region 20 is typically 50 to 200 mm, and preferably 70 to 150 mm, and the width is typically 10 to 80 mm, and preferably from 20 to 50 mm.

Compressed section 5 is a concave section formed by heat embossing treatment. Compressed section 5 in this embodiment is an example of the joint section for joining top sheet 2 and absorbent core 4 together. Joint sections at which top sheet 2 and absorbent core 4 are joined to each other may be formed by a bonding method other than heat embossing, such as, ultrasonic embossing, bonding with an adhesive, etc.

In heat embossing treatment, a predetermined section of the skin contact surface of top sheet 2 is compressed in the thickness direction of absorbent core 4 and is heated. Consequently, compressed section 5 is formed as a concave section which integrates top sheet 2 with absorbent core 4 in the thickness direction.

The heat embossing treatment is carried out by, for example, a method of embossing top sheet 2 and absorbent core 4 by passing them together between an embossing roll having an outer peripheral surface with protrusions and a flat roll having a smooth outer peripheral surface. In this method, heating the embossing roll and/or flat roll allows heating during compression. Protrusions of the embossing roll are provided so as to correspond to the shape, arrangement pattern, etc., of compressed groove 5. In the heat embossing treatment, the heating temperature is typically 80 to 180°C, and preferably 120 to 160°C, the pressure is 10 to 3000 N/mm, and preferably 50 to 500 N/mm, and the treating time is typically 0.0001 to 5 seconds, and preferably from 0.005 to 2 seconds.

By heat embossing treatment, the thermoplastic resin fibers contained in absorbent core 4 are thermally fused with the material which constitutes top sheet 2, and thereby top sheet 2 and absorbent core 4 are integrated together. Consequently, the interfacial peel strength between top sheet 2 and absorbent core 4 is increased.

The joining strength in dry state of compressed section 5 is preferably 1.53 N/25 mm or more, and the joining strength in wet state of compressed section 5 is preferably 0.95 N/25 mm or more. Consequently, sanitary napkin 1 can effectively prevent the interfacial delamination between top sheet 2 and absorbent core 4 from occurring and can effectively prevent the constituent fibers of absorbent core 4 (when the absorbent core also contains SAP particles, both the constituent fibers and SAP particles of the absorbent core) from leaking through top sheet 2 from the non-covered region resulting from the interfacial delamination, even if the strength of absorbent core 4 is decreased due to absorption of a liquid excrement. Therefore, sanitary napkin 1 is less likely to give uncomfortable feeling to the wearer due to the leakage of the fibers and SAP particles. Here, the weight ratio of the thermoplastic resin fibers to the water absorbent fibers of 1/9 or more is a necessary condition for achieving such a joining strength (particularly strength in wet state) for compressed section 5.

With respect to the joining strength of compressed section 5, "N/25 mm" means a joining strength (N) per 25 mm width in the extending direction of compressed section 5, and the extending direction of compressed section 5 incudes, for example, the lengthwise direction (conveyance direction (MD direction) during manufacturing) of sanitary napkin 1, the short direction ((CD direction) perpendicular to the MD direction) of sanitary napkin 1, etc., and the extending direction is preferably the lengthwise direction (MD direction) of sanitary napkin 1. Therefore, the joining strength in dry and wet states of compressed section 5 is preferably the joining strength in dry and wet states of the part of compressed section 5, extending in the lengthwise direction of sanitary napkin 1.

If the weight ratio of the thermoplastic resin fibers to the water absorbent fibers (thermoplastic fibers/water absorbent fibers) in absorbent core 4 is 1/9 to 5/5, it is possible to achieve a joining strength in dry state of 1.53 to 7.65 N/25 mm for compressed section 5 and a joining strength in wet state of 0.95 to 4.34 N/25 mm for compressed section 5.

Desired joining strengths in dry and wet states can be achieved by adjusting the weight ratio of the thermoplastic resin fibers to the water absorbent fibers in absorbent core 4 to 1/9 or more, and appropriately adjusting the conditions of heat embossing treatment, the type of the thermoplastic resin fibers in top sheet 2, the presence or absence of the thermoplastic resin fibers in top sheet 2, etc.

The joining strength in dry state of compressed section 5 can be measured in the following manner.

A sample piece (50 mm length × 25 mm width) under standard condition (under an atmosphere of a temperature of 20°C and a humidity of 60%) is attached to a tensile tester (for example, AG-1kNI manufactured by Shimadzu Corporation) by clamping the absorbent with a upper jaw and clamping the top sheet with a lower jaw at a clamping interval of 20 mm and is loaded at a tensile rate of 100 mm/min. until the absorbent and top sheet are completely peeled apart (the maximum load) to determine the joining strength (N/25 mm) of the compressed section. Herein, "N/25 mm" means a joining strength (N) per 25 mm width of the sample piece when the lengthwise direction of the sample piece is the tensile direction.

The joining strength in wet state of compressed section 5, can be measured in the following manner.

After immersing a sample piece (50 mm length × 25 mm width) in ion-exchanged water until it is settled down by its own weight or after sinking the sample piece in the water for 1 hour, the joining strength in wet state is measured in the same manner as described for the joining strength in dry state. Herein, "N/25 mm" means a joining strength (N) per 25 mm width of the sample piece when the lengthwise direction of the sample piece is the tensile direction.

Regarding the measurements of the joining strengths in dry and wet states, the measurement conditions described in ISO 9073-3 or JIS L 1913 6.3 are used for the measurement conditions other than those specified above.

The sample pieces used to measure the joining strength are cut from sanitary napkin 1 so as to include a part of compressed section 5. For example, the sample pieces are cut from sanitary napkin 1 so as to include a part of compressed section 5, extending in the lengthwise direction of sanitary napkin 1. The lengthwise direction of the sample pieces thus cut out preferably corresponds to the extending direction of compressed section 5. For example, sample pieces (for example, 50 mm length × 25 mm width) having a lengthwise direction corresponding to the extending direction of compressed section 5 can be prepared by cutting sanitary napkin 1 perpendicular to compressed section 5 extending in the lengthwise direction.

In view of further increasing the interfacial peel strength between top sheet 2 and absorbent core 4, top sheet 2 preferably contains one or more thermoplastic resin fibers.

The thermoplastic resin fibers contained in top sheet 2 is not particularly limited as long as the intersection points between fibers are heat fusible. The thermoplastic resin which constitutes the thermoplastic resin fibers includes, for example, polyolefins, polyesters, polyamides, etc.

The polyolefins include, for example, linear low density polyethylene (LLDPE), low density polyethylene (LDPE), medium density polyethylene (MDPE), high density polyethylene (HDPE), polypropylene, polybutylene, copolymers comprising mainly of these components (for example, ethylene-vinyl acetate copolymer (EVA), ethylene-ethyl acrylate copolymer (EEA), ethylene-acrylic acid copolymer (EAA), ionomer resins). Polyethylenes, particularly HDPE are preferred, since they have a relatively low softening point of about 100°C and therefore have excellent thermal processing properties as well as a low rigidity and flexible touch.

The polyesters include, for example, polyesters of a linear or branched chain polyhydroxy alkanoic acid having carbon atoms of up to 20, including polyethylene terephthalate (PET), poly(trimethylene terephthalate) (PTT), polybutylene terethalate (PBT), polylactic acid, polyglycolic acid, etc., and copolymers comprising mainly of these polyesters, or copolymerized polyesters formed by copolymerizing as a main component an alkylene terephthalate with a minor amount of other components. PET is preferred from the viewpoint of the capability of constituting fibers and nonwoven fabric having high cushioning properties due to having elastic resilience and from the economic viewpoint of industrial availability at a low cost.

The polyamides include, for example, 6-Nylon, 6,6-Nylon, etc.

Top sheet 2 and/or covering layer 42 may be comprised of one or more thermoplastic resin fibers, and may contain other fibers that are not heat-fusible with the thermoplastic resin fibers. Other fibers that are not heat-fusible with the thermoplastic resin fibers include, for example, regenerated fibers such as rayon, etc.; semi-synthetic fibers such as acetate, etc.; natural fibers such as cotton, wool, etc.; and synthetic fibers such as polypropylene, polyethylene, polyesters, nylons, polyvinyl chloride, vinylon, etc. The amount of other fibers that are not heat-fusible with the thermoplastic resin fibers is typically 5 to 70 wt%, and preferably 10 to 30 wt% of top sheet 2 and covering layer 42.

The form of the thermoplastic resin fibers contained in top sheet 2 includes, for example, core-sheath type, side-by-side type, islands/sea type, etc.
In view of thermal adhesive properties, conjugate fibers are preferably composed of a core part and a sheath part. The cross sectional shape of the core in the sheath-core type conjugate fibers includes, for example, circular, triangular type, square type, star-shaped, etc., and the core part may be a hollow or may be porous. The cross-sectional area ratio of the core/sheath structure is not particularly limited, but is preferably 80/20 to 20/80, and more preferably 60/40 to 40/60.

The thermoplastic resin fibers contained in top sheet 2, may be imparted with a three-dimensional crimped shape. Consequently, even if the fiber orientation is aligned to the planar direction, the buckling strength of the fibers is exerted in the thickness direction, thereby making the fibers harder to crush even if an external force is applied thereto.

The three-dimensional crimped shape includes, for example, a zigzag shape, and a Omega shape, a spiral shape, etc., and the method for imparting a three-dimensional crimped shape includes, for example, mechanical crimping, shaping by heat shrinking, etc. Mechanical crimping can be controlled by the peripheral speed difference in line speed, heat, pressurization, etc., with respect to continuous linear fibers after spinning, and the greater the number of crimps per unit length of the crimped fibers, the greater the buckling strength of the fibers under external pressure. The number of crimps is typically 10 to 35 per inch, and preferably 15 to 30 per inch. Shaping by heat shrinking can provide a three-dimensional crimping by using the difference in heat shrinking resulted from the melting temperature difference by, for example, heating a fiber comprising two or more resins having different melting points. The cross-sectional shapes of the fibers include, for example, eccentric type, side-by-side type of core-sheath type conjugate fibers. Such fibers have a heat shrinking rate of preferably 5 to 90%, and more preferably 10 to 80%.

Absorbent core 4 may be densified by ejecting a high-pressure steam to a mixed material comprising water absorbent fibers and thermoplastic resin fibers (in a preferred embodiment, water absorbent fibers, thermoplastic resin fibers and SAP particles). The fiber density of absorbent core 4 can be adjusted to a desired range by densification by means of ejecting of a high-pressure steam. Upon ejecting of a high-pressure steam to the mixed material, the steam is penetrated into the mixed material, thereby cleaving hydrogen bonds (for example, hydrogen bonds among water absorbent fibers, hydrogen bonds among thermoplastic resin fibers, hydrogen bonds among water absorbent fibers-thermoplastic resin fibers, etc.) to soften the mixed material. Accordingly, the pressure required for the densification is decreased, and the density of the softened mixed material can be easily adjusted. Reforming of hydrogen bonds by drying the mixed material having an adjusted density suppresses the elastic recovery of fibers (increase in bulkiness), thereby retaining the fiber density of absorbent core 4 within a specific range.

Densification by means of ejecting of a high-pressure steam is particularly preferred when a unsaturated carboxylic acid anhydride (for example, maleic anhydride or derivatives thereof) is contained in the thermoplastic resin fibers as a monomer component. If the unsaturated carboxylic acid anhydride groups contained in the thermoplastic resin fibers are converted to unsaturated carboxylic acid groups by the reaction with water vapor, the number of oxygen atoms capable of forming hydrogen bonds is increased, and therefore the elastic recovery (increase in bulkiness) of the densified fibers is suppressed effectively.

Densification by means of ejecting of a high-pressure steam is carried out, for example, after adhesion of the thermoplastic resin fibers to the water absorbent fibers. The temperature, steam pressure, etc., of the high-pressure steam can be adjusted appropriately in accordance with the density range required, etc. The temperature of the high-pressure steam is preferably less than the melting temperature of the thermoplastic resin fibers (for example, the melting point of the sheath component, if the thermoplastic resin fibers are core-sheath type conjugate fibers). The high-pressure steam is jetted at 0.03 kg/m$^2$ to 1.23 kg/m$^2$ per unit surface area. The steam pressure of the high-pressure steam is typically 0.1 to 2 Mpa, and preferably 0.3 to 0.8 Mpa.

When the densification by means of ejecting of a high-pressure steam is carried out, the fiber basis weight of absorbent core 4 is preferably 40 to 900 g/m$^2$, and more preferably 100 to 400 g/m$^2$. If the fiber basis weight is less than 40 g/m$^2$, the fiber amount is too small, and thereby rendering the densification by means of a high-pressure steam difficult, whereas if the fiber basis weight is more than 900 g/m$^2$, the fiber amount is too much, and thereby rendering the internal penetration difficult.

By ejecting a high-pressure steam, it is possible to form ridges and grooves on a surface of absorbent core 4. The numbers of ridges and grooves, the spacing, etc., vary with the number, pitch, etc., of the nozzles for ejecting the high pressure steam. The sections to which a high-pressure steam is jetted become grooves. The ridges and grooves may be formed on the side of top sheet 2 of absorbent core 4 or may be formed on the side of back sheet 3 of absorbent core 4.

The ridges and grooves may be formed so as to extend in the longitudinal direction (Y-axis direction) of sanitary napkin 1 and be alternately arranged in the width direction (X-axis direction) of sanitary napkin 1. The ridges and grooves may extend continuously in the longitudinal direction (Y-axis direction) of sanitary napkin 1 or may extend intermittently in the longitudinal direction with sections having no ridge or groove. For example, the ridges and grooves may extend intermittently so that the sections which do not form ridge or groove have a shape such as a rectangular-shape in plan view, staggered-shape in plan view, etc.

Also, the ridges and grooves may be formed so as to extend in the width direction (X-axis direction) of sanitary napkin 1 and be alternately arranged in the longitudinal direction (Y-axis direction) of sanitary napkin 1. The ridges and grooves may extend continuously in the width direction (X-axis direction) of sanitary napkin 1 or may extend intermittently in the width direction with sections having no ridge or groove. For example, the ridges and grooves may extend intermittently so that the sections which do not form ridge or groove have a shape such as a rectangular-shape in plan view, staggered-shape in plan view, etc. When a plurality of ridges and grooves extending in the width direction (X-axis direction) of sanitary napkin 1 are formed, absorbent core 4 hardly causes twisting and is easily deformable to a curved shape along the shape of the body of the wearer, even if a force is applied to absorbent core 4 in the width direction. Therefore, it hardly gives an uncomfortable feeling to the wearer.

The shape of the ridges is not particularly limited. For example, the top and side surfaces of the ridges are curved, and the cross-sectional shape of the ridges is a substantially inverted U-shape toward the top sheet or back sheet. The cross-sectional shape of the ridges may be appropriately modified, and may be, for example, a dome shape, trapezoidal shape, triangular shape, $\Omega$-shaped square shape, etc. The width of the ridges decreases from the bottom to the top so as to maintain the space of the grooves even if a force is applied to absorbent core 4 and the ridges are crushed.

In view of transferring properties of liquid from top sheet 2, the width of the ridges is preferably 0.5 to 10 mm, and more preferably 2 to 5 mm. From the same viewpoint, the width of the grooves is preferably 0.1 to 10 mm, and more preferably 1 to 5 mm.

If a plurality of ridges is formed, the width of the ridges may be substantially the same or may be different. For example, a plurality pf ridges may be formed so that the width of one of the ridges is different from the width of another ridge and is substantially the same as the width of a still another ridge. The same is true when a plurality of grooves is formed.

A high pressure steam may be jetted over the entire of the mixed material or may be jetted to a part of the mixed material. In addition, the temperature, steam pressure, etc., of the high pressure steam to be jetted may be varied part by part of the mixed material. The fiber density distribution of absorbent core 4 can be varied by partially ejecting a high pressure steam to the mixed material, or by varying the temperature, steam pressure, etc. of the high pressure steam to be jetted part by part of the mixed material.

A high pressure steam may be jetted while pressing the mixed material, or a high pressure steam may be jetted without pressing the mixed material. The fiber density distribution of absorbent core 4 can be varied by ejecting a high pressure steam to the mixed material while pressing a part of the mixed material and ejecting a high pressure steam to the mixed material without pressing the other parts of the mixed material. For example, the fiber density distribution can be varied by ejecting a high pressure steam to the mixed material while passing the mixed material between mesh conveyor belts having openings to apply the high pressure steam directly to the mixed material without pressing at the parts of the openings of the mesh conveyor belt and to apply the high pressure steam to the mixed material while being pressed at the non-opening parts of the mesh conveyor belt.

As compared to other methods, the densification by ejecting a high pressure steam is advantageous in the following points. If the mixed material is densified by press roll molding, a high compression is required to impart an inter-fiber bonding force prevailing over the repulsive force of the fibers. In addition, even if the fibers are once compressed by high compression, the fibers are elastically recovered, and thereby the bulkiness is returned to the original one. On the other hand, when the mixed material is densified by a combination of a press roll and water spraying, water can be penetrated into the inside of the mixed material if the basis weight is 100 g/m$^2$ or less, whereas it is difficult to penetrate water into the inside of the mixed material if the basis weight is more than 100 g/m$^2$, and thereby it is difficult to form hydrogen bonds within the mixed material. In addition, although the application of an excess of water allows water to penetrate into the inside of the mixed material, an excess quantity of heat and time are required to evaporate water, thereby resulting in a reduction in productivity. In contrast, the densification is carried out by ejecting of a high pressure steam, steam is penetrated into the inside of the mixed material, and thereby hydrogen bonds (e.g., hydrogen bonds formed between the water absorbent fibers, between the thermoplastic resin fibers, and between the water absorbent fibers and thermoplastic fibers, etc.) are cleaved and the mixed material is softened. Accordingly, the pressure required for the densification is decreased, and thereby the softened mixed material is easily adjusted in its density. In addition, the productivity is improved, since steam is easily evaporated, and thereby the time required for drying is short.

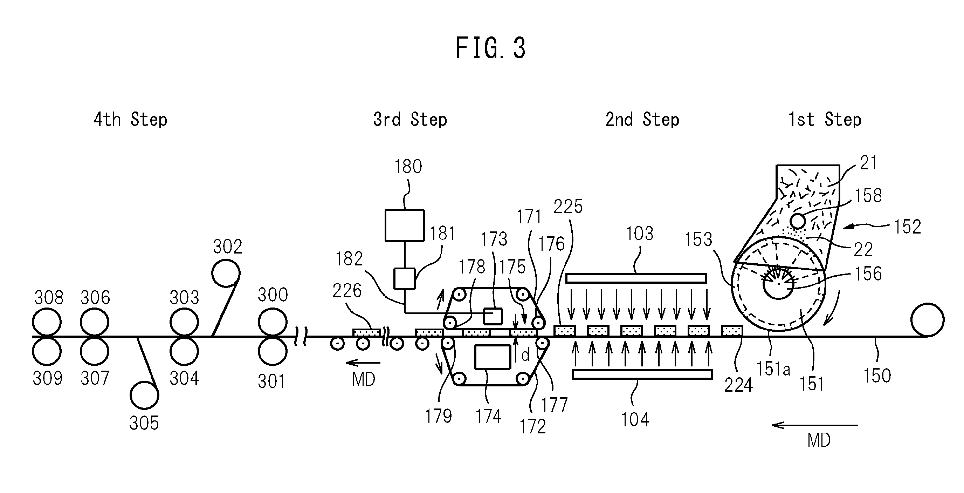

Specific examples of the steps for manufacturing sanitary napkin 1 will be described with reference to Fig. 3.

[First Step]

Peripheral surface 151a of suction drum 151 which rotates in the conveyance direction MD have recessed parts 153 formed thereon at a predetermined pitch in the circumferential direction as molds for filling an absorbent material. When suction drum 151 rotates and recessed parts 153 enter into material feeding unit 152, suction unit 156 acts on recessed parts 153 and the absorbent material supplied from material feeding unit 152 is vacuum sucked into recessed parts 153.

Hooded material feeding unit 152 is formed so as to cover suction drum 151, and material feeding unit 152 feeds mixed material 21 of cellulose-based water absorbent fibers and thermoplastic resin fibers to recessed parts 153 by air conveyance. In addition, material feeding unit 152 provides with particle feeding unit 158 for feeding superabsorbent polymer particles 22 and supplies superabsorbent polymer particles 22 to recessed parts 153. The cellulose-based water-absorbing fibers, thermoplastic resin fibers and superabsorbent polymer particles are fed to recessed parts 153 in a mixed state, absorbent material layers 224 are formed in recessed parts 153. Absorbent material layers 224 formed in recessed parts 153 are transferred onto carrier sheet 150 that travels toward the conveyance direction MD.

[Second step]

Absorbent material layers 224 transferred onto carrier sheet 150 are transported away from peripheral surface 151a of suction drum 151 in the conveyance direction MD. Absorbent material layers 224 in uncompressed state are arranged intermittently on carrier sheet 150 in the conveyance direction MD. Heating section 103 blows air heated to 135°C to the upper surface of absorbent material layer 224 at an air flow of 5 m/sec., and heating section 104 blows air heated to 135°C to the lower surface of absorbent material layer 224 at an air flow of 5 m/sec. Consequently, the thermoplastic resin fibers contained in absorbent material layer 224 melts, and thereby form absorbent material layer 225 in which thermoplastic resin fibers are bonded (thermally fused) to each other, thermoplastic resin fibers and pulps are bonded (thermally fused) to each other, and thermoplastic resin fibers and superabsorbent polymer particles are bonded (thermally fused) to each other. The conditions (temperature, wind velocity, heating time) of the heated air blown to absorbent material layer 224 are controlled appropriately in accordance with the production speed, etc.

[Third Step]

Vertically paired air-permeable mesh conveyor belts 171, 172 serve to transport absorbent material layers 225 on carrier sheet 150 in the machine direction MD, while compressing absorbent material layer 225. The dimension d in the vertical direction at parallel travelling unit 175 (the distance between mesh conveyor belt 171 and 172) are set to have a predetermined value by adjusting the gap between upstream-side upper roll 176 and upstream-side lower roll 177 which rotate in the conveyance direction MD as well as the gap between downstream-side upper roll 178 and downstream-side lower roll 179, and absorbent material layers 225 are compressed by mesh conveyor belts 171, 172 to have a predetermined thickness. In Fig. 3, steam ejection unit 173 and steam suction unit 174 are disposed in parallel travelling unit 175 so as to face with each other across mesh conveyor belts 171, 172. In steam ejection unit 173, nozzles (not shown) having a diameter of, for example, 0.1 to 2 mm, are disposed in the cross direction CD (not shown) orthogonal to the machine direction MD and the thickness direction TD at a pitch of 0.5 to 10 mm, preferably 0.5 to 5 mm, and more preferably 0.5 to 3 mm, across absorbent material layer 225, and the respective nozzles are supplied via piping 182 with a high-pressure steam having a temperature equal to or above the boiling point of water, which is produced at steam boiler 180 and is regulated to have a steam pressure of, for example, 0.1 to 2.0 MPa by pressure control valve 181. A high-pressure steam is ejected from the respective nozzles to absorbent material layers 225 held in compressed state by mesh conveyor belts 171, 172 through mesh conveyor belt 171. The amount of the high-pressure steam ejected to absorbent material layer 225 is adjusted in accordance with the travelling speed of mesh conveyor belts 171, 172, and is preferably ejected to absorbent material layers 225 facing mesh conveyor belt 171 in the range of 1.23 kg/m$^2$ to 0.03 kg/m$^2$ when mesh conveyor belts 171, 172 travel at 5 to 500 m/min. Steam passes mesh conveyor belt 171, absorbent material layers 225, and mesh conveyor belt 172, in this order in the thickness direction of absorbent material layers 225, and is recovered under the vacuum pressure suction effect of steam suction unit 174. Absorbent material layers 225 to which a high-pressure steam has been applied travel in the conveyance direction MD and are separated from mesh conveyor belts 171, 172, and then are forwarded to the fourth step. Ridges and grooves are formed on the surface of absorbent material layers 225 to which a high-pressure steam has been applied. The numbers, intervals, etc., of the ridges and grooves can be adjusted by the number, pitch, etc., of steam ejection unit 173. The sections to which a high-pressure steam has been applied form grooves.

In the third step, at least one of mesh conveyor belts 171, 172 has a sufficient flexibility to be easily deformed in the thickness direction TD to prevent absorbent material layers 225 from being locally compressed by mesh conveyor belts 171, 172. Metallic wire mesh belts formed of stainless alloys, bronze, etc., and plastic mesh belts formed of polyester fibers, aramid fibers, etc., may be used in mesh conveyor belts 171, 172, and metallic belts formed of a perforated metal plate may be used in place of the mesh belts. If absorbent material layer 225 is extremely incompatible with contamination of a metal powder, it is preferable to use a plastic mesh belt. In addition, if a plastic mesh belt is required to have a high heat resistance, it is preferable to use a mesh belt made of poly(phenylene sulfide) resin. A plain-woven mesh belt having 10 to 75 mesh using poly(phenylene sulfide) resin has flexibility, and is an example of particularly preferred mesh belts that can be used as either mesh conveyor belt 171 or mesh conveyor belt 172. Steam ejection unit 173 and piping182 may be preferably provided with an appropriate thermal insulation or a drain discharge system. Such countermeasures can prevent absorbent material layer 225 from excessively containing water due to the ejection of the drain generated in the steam ejection unit 173, etc., from the nozzles. The steam which is ejected to absorbent material layers 225 is in the form of dry steam containing no moisture liquid, in the form of saturated steam, or in the form of wet steam containing liquid. If the steam is wet steam or saturated steam, a pulp can be easily transformed into a wet state and deformed. Dry steam can vaporize the moisture contained in a pulp, and the vaporized moisture can facilitate the deformation of the pulp. If the pulp is a thermoplastic synthetic fiber, the heat of the dry steam can facilitate the deformation of the thermoplastic synthetic fiber. Steam ejection unit 173 may be provided with a heating system to transform steam into superheated steam and to eject the superheated steam. Steam suction unit 174 is preferably provided with a piping through which the sucked high pressure steam may be forwarded to an exhaust blower (not shown) after passing through a steam-water separator. It should be appreciated here that the present invention may be implemented in an embodiment in which the positions of steam ejection unit 173 and steam suction unit 174 are interchanged, i.e., steam ejection unit 173 and steam suction unit 174 are placed upside down. If it is not necessary to collect the high pressure steam, the present invention may be implemented without the provision of steam suction 174.

If it is not necessary to implement the densification by the ejection of a high-pressure steam, the third step may be omitted.

[Fourth Step]

The fourth step is an example of a step of manufacturing a typical sanitary napkin. In this step, a pair of rolls 300, 301 cut out absorbent material layer 226 obtained in the third step (if the third step is omitted, absorbent material layer 225 obtained in the second step) into a predetermined shape to form an absorbent core. A top sheet is supplied from roll 302 and is sealed by heat embossing 303, 304 having a high compression section and a low compression section, and thereby the top sheet and absorbent core are integrated with each other. Then, a back sheet is supplied from roll 305, and absorbent core 4 passes through steps 306, 307 for sealing the product periphery with heat embossing while absorbent core 4 being sandwiched between the top sheet and the back sheet and finally cut into a product shape by steps 308, 309.

EXAMPLES

The present invention will be described in more detail with reference to production examples and experimental examples, and the scope of the present invention is not limited to the production examples and experimental examples.

The heat-fusible conjugate fibers and superabsorbent polymer particles used in the production example are as follows.

[Heat-fusible conjugate fibers A]

As heat-fusible conjugate fibers A (hereinafter referred to as "conjugate fibers A"), core-sheath type conjugate fibers comprising as a core component polyethylene terephthalate (PET) and as a sheath component a high-density polyethylene (HDPE) having a maleic anhydride-containing vinyl polymer graft-polymerized thereto were used. Conjugate fibers A have a core-sheath ratio of 50:50 (weight ratio), a titanium oxide content in the core component of 0.7 wt%, a fineness of 2.2 dtex, and a fiber length of 6 mm.

[Heat-fusible conjugate fibers B]

As heat-fusible conjugate fibers B (hereinafter referred to as "conjugate fibers B"), core-sheath type conjugate fibers comprising as a core component polyethylene terephthalate (PET) and as a sheath component a common high-density polyethylene (HDPE) were used. Conjugate fibers B have a core-sheath ratio of 50:50 (weight ratio), a titanium oxide content in the core component of 0.7 wt%, a fineness of 2.2 dtex, and a fiber length of 6 mm.

[Superabsorbent resin particles]

As superabsorbent resin particles (hereinafter referred to as "SAP particles"), particles of polyacrylic acid salt crosslinked product (manufacturer Sumitomo Seika Chemicals Co., Ltd.) were used. In the particle size distribution of the SAP particles, particles of 150 to 250 $\mu$m constitute 3.9%, particles of 250 to 300 $\mu$m constitute 5.3%, particles of 300 to 355 $\mu$m constitute 17.1%, particles of 355 to 500 $\mu$m constitute 56.3%, particles of 500 to 600 $\mu$m constitute 11.2%, particles of 600 to 710 $\mu$m constitute 4.9%, particles of 710 to 850 $\mu$m constitute 1.2%, and particles of more than 850 $\mu$m constitute 0.1%.

The measurement methods for the basis weight, thickness and density of the absorbent core were carried our as follows.

[Basis weight]

The measurement on the basis weight ($g/m^2$) of the absorbent core was carried out as follows.

Three 100 mm $\times$ 100 mm sample pieces were cut out from the absorbent core and the weight was measured for each sample piece under standard condition (temperature 23 $\pm$ 2°C, relative humidity 50 $\pm$ 5%) with a direct reading balance (Electronic Balance HF-300 manufactured by Kensei Co., Ltd.), and the weight per unit surface area ($g/m^2$) of

the absorbent core calculated from the average value of the three measurement values was determined as the basis weight of the absorbent core.

Regarding the measurement on the basis weight of the absorbent core, the measurement conditions described in ISO 9073-1 or JIS L 1913 6.2 were used for the measurement conditions other than those specified above.

[Thickness]

The measurement on the thickness (mm) of the absorbent core was carried out as follows.

The thicknesses of five different points of the absorbent core under standard condition (temperature $23 \pm 2°C$, relative humidity $50 \pm 5\%$) were measured with a thickness gauge (for example, FS-60DS manufactured by Daiei Kagaku Seiki Manufacturing Co., Ltd., measurement surface 44 mm (diameter), measurement load 3 $g/cm^2$) by loading at a constant pressure of 3 $g/cm^2$ and measuring the thickness after 10 seconds pressure loading at each point, and an average value of the five measurement values is determined as the thickness of the absorbent core.

[Density]

Density of the absorbent core was calculated on the basis of the following equation.

$$D \; (g/cm^3) \; = \; B \; (g/m^2) \; / \; T \; (mm) \; \times \; 10^{-3}$$

wherein D, B and T represent the density, the basis weight and thickness of the absorbent core, respectively.

In the following Production Examples I and experimental Examples I, the fiber materials and absorbent cores produced using conjugate fibers A will be referred to as "fiber materials A" and "absorbent cores A", respectively, and the fiber materials and absorbent cores produced using conjugate fibers B will be referred to as "fiber materials B" and "absorbent cores B", respectively. In addition, the absorbent cores produced using fiber materials A1 to A8 having different mass mixing ratios of conjugate fibers A with respect to a pulp will be referred to as "absorbent cores A1 to A8", respectively, and the absorbent cores produced using fiber materials B1 to B8 having different mass mixing ratios of conjugate fibers B with respect to a pulp will be referred to as "absorbent cores B1 to B8", respectively.

In the following Production Examples II and experimental Examples II, the fiber materials and absorbent cores produced using conjugate fibers A will be referred to as "fiber materials C" and "absorbent cores C", respectively, and the fiber materials and absorbent cores produced using conjugate fibers B will be referred to as "fiber materials D" and "absorbent cores D", respectively. In addition, the absorbent cores produced using fiber materials C1 to C5 having different mass mixing ratios of conjugate fibers A with respect to a pulp will be referred to as "absorbent cores C1 to C5", respectively, and the absorbent cores produced using fiber materials D1 to D6 having different mass mixing ratios of conjugate fibers B with respect to a pulp will be referred to as "absorbent cores D1 to D6", respectively.

[Production Example I-1] Production of Absorbent Cores A (A1 to A8) , B (B1 to B8)

(1) Production of fiber materials A (A1 to A8)

Fiber materials A1 to A8 (basis weight: 200 $g/m^2$) were produced by mixing and stacking a pulp (NB416 manufactured by Wear Hauser Inc.) and conjugate fibers A at a weight ratio of pulp: conjugate fibers A = 9.5:0.5 (A1), 9:1 (A2), 8:2 (A3), 6.5:3.5 (A4), 5:5 (A5), 3.5:6.5 (A6), 2:8 (A7) , and 0:10 (A8).

(2) Production of fiber materials B (B1 to B8)

Fiber materials B1 to B8 (basis weight: 200 $g/m^2$) were produced by mixing and stacking a pulp (NB416 manufactured by Wear Hauser Co.) and conjugate fibers B at a weight ratio of pulp: conjugate fibers B = 10:0 (B1), 9:1 (B2), 8:2 (B3), 6.5:3.5 (B4), 5:5 (B5), 3.5:6.5 (B6), 2:8 (B7), and 0:10 (B8).

(3) Productions of absorbent cores A (A1 to A8), B (B1 to B8)

Absorbent cores A1 to A8 and B1 to B8 were produced by bonding fiber materials A1 to A8 and B1 to B8 in a common through-air method, and thermally fusing conjugate fibers A and B. In this case, the heating temperature was set to 135°C, the air flow was set to 5 m/sec, and the heating time was set to 20 seconds.

The compositions and physical properties of absorbent cores A1 to A8, B1 to B8 will be shown in Table 1.

[Table 1]

| | | Composition | | | Physical properties after heat treatment | | |
|---|---|---|---|---|---|---|---|
| | | Pulp basis weight (g/m$^2$) | Conjugate fiber basis weight (g/m$^2$) | Mass mixing ratio (Pulp : Conjugate fibers) | Actual basis weight (g/m$^2$) | Thickness (mm) | Density (g/cm$^3$) |
| Absorbent Cores | A1 | 190 | 10 | 9.5:0.5 | 203 | 8.68 | 0.0234 |
| | A2 | 180 | 20 | 9:1 | 210 | 8.92 | 0.0235 |
| | A3 | 160 | 40 | 8:2 | 197 | 8.76 | 0.0225 |
| | A4 | 130 | 70 | 6.5:3.5 | 206 | 9.53 | 0.0216 |
| | A5 | 100 | 100 | 5:5 | 215 | 9.27 | 0.0232 |
| | A6 | 70 | 130 | 3.5:6.5 | 219 | 8.54 | 0.0256 |
| | A7 | 40 | 160 | 2:8 | 223 | 6.87 | 0.0325 |
| | A8 | 0 | 200 | 0:10 | 222 | 6.47 | 0.0344 |
| | B1 | 200 | 0 | 10:0 | - | - | - |
| | B2 | 180 | 20 | 9:1 | 205 | 8.66 | 0.0237 |
| | B3 | 160 | 40 | 8:2 | 206 | 8.21 | 0.0251 |
| | B4 | 130 | 70 | 6.5:3.5 | 215 | 9.07 | 0.0237 |
| | B5 | 100 | 100 | 5:5 | 214 | 9.02 | 0.0237 |
| | B6 | 70 | 130 | 3.5:6.5 | 222 | 9.07 | 0.0245 |
| | B7 | 40 | 160 | 2:8 | 223 | 7.97 | 0.0280 |
| | B8 | 0 | 200 | 0:10 | 218 | 5.63 | 0.0387 |

[experimental Example I-1] Measurements on the fiber falling-off rates for absorbent cores A (A1 to A5), B (B2 to B5)

(1) Measurement Methods

Sample pieces (100mm length × 100 mm width) were prepared by cutting absorbent cores A1 to A5 and B2 to B5 produced in Production Example I-1 and were used in the measurements on the fiber falling-off rates in dry and wet states.

[Fiber falling-off rate in dry state (%)]

An empty 2000 mL vessel (JP-2000 manufactured by NIKKO Co., Ltd.) was charged with pre-test sample pieces (100 mm × 100 mm) and was shaken with a shaker (SHKV-200 manufactured by IWAKI, Co., Ltd.) at a shaking rate of 300 rpm for 1 hour, and a sample piece which maintained a sheet form after shaking was removed from the vessel and was marked as a post-test sample piece. Then, a fiber falling-off rate (%) (the weight of the fallen fibers/the weight of the pre-test sample piece × 100) was calculated based on the weight of the fallen fibers (the weight of the pre-test sample piece minus the weight of the post-test sample piece).

[Fiber falling-off rate in wet state (%)]

A 2000 mL vessel (JP-2000 manufactured by NIKKO Co., Ltd.) containing 1000 mL of distilled water was charged with pre-test sample pieces (100 mm × 100 mm) and was shaken with a shaker (SHKV-200 manufactured by IWAKI, Co., Ltd.) at a shaking rate of 250 rpm for 30 seconds, and a sample piece which maintained a sheet form after shaking was removed from the vessel, was dried at 80°C for 12 hours or more using an air-blowing, constant-temperature thermostat (DNE-910 manufactured by Yamato Scientific Co., Ltd.), and was marked as a post-test sample piece. Then, a fiber falling-off rate (%) (the weight of the fallen fibers/the weight of the pre-test sample piece $\times$ 100) was calculated based on the weight of the fallen fibers (the weight of the pre-test sample piece minus the weight of the post-test sample piece).

(2) Results and observation

The measurement results will be shown in Tables 2 and 3.

[Table 2]

| | | Composition | In dry state | | | |
|---|---|---|---|---|---|---|
| | | Mass mixing ratio (Pulp : Conjugate fibers) | Pre-test weight (g) | Post-test weight (g) | Weight of fallen fibers (g) | Fiber falling-off rate (%) |
| Absorbent cores | A1 | 9.5:0.5 | 2.01 | 1.91 | 0.10 | 5.0 |
| | A2 | 9:1 | 2.15 | 2.09 | 0.06 | 2.8 |
| | A3 | 8:2 | 2.12 | 2.09 | 0.03 | 1.5 |
| | A4 | 6.5:3.5 | 2.05 | 2.02 | 0.03 | 1.3 |
| | A5 | 5:5 | 2.11 | 2.09 | 0.02 | 0.9 |
| | B2 | 9:1 | 1.99 | 1.90 | 0.09 | 4.5 |
| | B3 | 8:2 | 2.01 | 1.94 | 0.07 | 3.5 |
| | B4 | 6.5:3.5 | 2.03 | 1.98 | 0.05 | 2.5 |
| | B5 | 5:5 | 2.19 | 2.15 | 0.04 | 1.9 |

[Table 3]

| | | Composition | In wet state | | | |
|---|---|---|---|---|---|---|
| | | Mass mixing ratio (Pulp : Conjugate fibers) | Pre-test weight (g) | Post-test weight (g) | Weight of fallen fibers (g) | Fiber falling-off rate (%) |
| Absorbent cores | A1 | 9.5:0.5 | 2.01 | 0.22 | 1.80 | 89.3 |
| | A2 | 9:1 | 2.03 | 1.76 | 0.27 | 13.3 |
| | A3 | 8:2 | 1.96 | 1.71 | 0.25 | 12.8 |
| | A4 | 6.5:3.5 | 2.05 | 1.79 | 0.26 | 12.7 |
| | A5 | 5:5 | 2.02 | 2.00 | 0.02 | 1.0 |
| | B2 | 9:1 | 1.99 | 0.39 | 1.60 | 80.3 |
| | B3 | 8:2 | 2.01 | 0.87 | 1.14 | 56.7 |
| | B4 | 6.5:3.5 | 2.03 | 1.74 | 0.30 | 14.5 |
| | B5 | 5:5 | 2.19 | 1.98 | 0.21 | 9.4 |

Observation based on Table 2 and Table 3 are as follows.

Comparison among absorbent cores A1 to A5 shows that the greater the mass mixing ratio of conjugate fibers A to pulp, the lower the fiber falling-off rates in dry and wet states. Specifically, the fiber falling-off rate in dry state was significantly decreased from 5.0% (absorbent core A1) to 2.8% (absorbent core A2) and the fiber falling-off rate in wet state was significantly decreased from 89.3% (absorbent core A1) to 13.3% (absorbent core A2) with the increase in the mass mixing ratio of conjugate fibers A to the pulp from 0.5/9.5 (absorbent core A1) to 1/9 (absorbent core A2). Namely, the mass mixing ratio of conjugate fibers A to pulp of 1/9 has a critical significance in that the fiber falling-off rates in dry and wet states (especially fiber falling-off rate in wet state) when the mass mixing ratio is less than 1/9 are significantly different from the fiber falling-off rates in dry and wet states when the mass mixing ratio is equal to or more than 1/9. Therefore, in view of decreasing the fiber falling-off rates in dry and wet states, it is advantageous to set the mass mixing ratio of conjugate fibers A to pulp to 1/9 or more, and thereby a fiber falling-off rate in dry state of 2.8% or less and a fiber falling-off rate in wet state of 13.3% or less can be achieved. In addition, it is estimated that, if the mass mixing ratio of conjugate fibers A to pulp is 0.5/9.5 (absorbent core A1), the fiber number of conjugate fibers A is too low, and therefore a network of fibers is not sufficiently formed in conjugate fibers A, resulting in a large fiber falling-off rates in dry and wet states.

Also, comparison between absorbent cores (i.e., A2 and B2, A3 and B3, A4 and B4, A5 and B5) having the same mass mixing ratio of conjugate fibers A, B to pulp shows that absorbent cores A have fiber falling-off rates in dry and wet states lower than those of absorbent cores B at any mass mixing ratio. Therefore, when the mass mixing ratio of conjugate fibers A to pulp is the same as the mass mixing ratio of conjugate fibers B to pulp, the fiber falling-off rates in dry and wet states can be reduced in case where conjugate fibers A are used, as compared with the case where conjugate fibers B are used. In addition, if certain fiber falling-off rates in dry and wet states are desired to achieve, the mass mixing ratio of conjugate fiber to pulp can be reduced in case where conjugate fibers A is used, as compared with the case where conjugate fibers B are used (i.e., the mass mixing ratio is increased accordingly, thereby improving the absorbing performance).

[Production Example I-2] Productions of absorbent articles A (A1 to A5), B (B2 to B5)

A hot-melt adhesive (HMA) was applied to the air-blown surface of an air-through nonwoven fabric (basis weight: 30 g/m$^2$, size: 100 mm length (MD direction) $\times$ 80 mm width (CD direction)) with a spiral spray gun (manufactured by Nordson Corporation) at a basis weight of 5 g/m$^2$, and subsequently each of absorbent cores A1 to A5 and B2 to B5 (basis weight: 200 g, size: 100 mm length (MD direction) $\times$ 80 mm width (CD direction)) was laminated onto the air-blown surface. Subsequently, embossed sections at which the nonwoven fabric and the absorbent core are partially joined with each other were formed by heat embossing treatment to produce absorbent articles A1 to A5 and B2 to B5.

The heat embossing treatment was carried out using an embossing plate (heating temperature 110°C) having a convex section formed thereon as a plate of the nonwoven fabric side (upper side), and a plane plate (heating temperature 110°C) as a plate of the absorbent core sample side (lower side). The embossing treatment time was 3 seconds, and embossing pressure was 5 MPa (5 kPa/mm$^2$). By heat embossing treatment, an embossed section was formed extending in the longitudinal direction of the absorbent article when the absorbent article is viewed in plan view from the side of the nonwoven fabric. The embossed section included a low-compressed part and a high-compressed part, and the low-compressed part has a surface area of 803.01 mm$^2$ and the high-compressed part has a surface area of 188.65 mm$^2$.

[Experimental Example I-2] Measurements on joining strength of embossed sections for absorbent articles A (A1 to A5), B (B2 to B5)

(1) Measurement methods

Absorbent articles A1 to A5, B2 to B5 produced in Production Example I-2 were respectively cut perpendicular to the embossed section to prepare five sample pieces (50mm length $\times$ 25mm width), and the samples were used to measure the embossed section joining strength (N/25 mm).

[Embossed section joining strength in dry state (N/25 mm)]

A sample piece under standard condition (under an atmosphere of temperature 20°C and humidity 60%) was attached to the tensile testing machine (AG-1kNI manufactured by Shimadzu Corporation) at a gripping interval of 20 mm with attaching the absorbent core to an upper grip and attaching the nonwoven fabric to a lower grip. The joining strength (N) of the embossed section per width of 25 mm of the sample piece was measured at a tensile speed 100 mm/min., in the lengthwise direction of the sample piece as the tensile direction while applying a load until the nonwoven fabric and absorbent core are completely peeled apart (maximum load).

[Embossed section joining strength in wet state (N/25 mm)]

After immersing a sample piece in ion-exchanged water until it is settled down by its own weight or after sinking the sample piece in the water for 1 hour, the joining strength (N) of the embossed section per 25 mm width in the lengthwise direction of the sample piece was measured in the same manner as described above.

Regarding the measurements of the embossed section joining strengths in dry and wet states, the measurement conditions described in ISO 9073-3 or JIS L 1913 6.3 were used for the measurement conditions other than those specified above.

(2) Results and observation

The measurement results will be shown in Table 4.

[Table 4]

|  |  | Composition of absorbent core | Embossed section joining strength (N/25 mm) | |
| --- | --- | --- | --- | --- |
|  |  | Mass mixing ratio (Pulp : Conjugate fibers) | In dry state | In wet state |
| Absorbent articles | A1 | 9.5:0.5 | 0.99 | 0.73 |
|  | A2 | 9:1 | 1.53 | 0.95 |
|  | A3 | 8:2 | 3.95 | 2.24 |
|  | A4 | 6.5:3.5 | 4.51 | 2.91 |
|  | A5 | 5:5 | 7.65 | 4.34 |
|  | B2 | 9:1 | 0.96 | 0.75 |
|  | B3 | 8:2 | 1.76 | 1.71 |
|  | B4 | 6.5:3.5 | 2.37 | 1.99 |
|  | B5 | 5:5 | 3.69 | 3.24 |

Observation based on Table 4 is as follows.

An embossed section joining strength of less than 0.75 N/25 mm may lead to occurrence of the interfacial delamination between the top sheet and the absorbent core during the use of the absorbent article. Accordingly, when both embossed section joining strengths in dry and wet states should be 0.75 N/25 mm or more, the absorbent articles that satisfy this requirement are absorbent articles A2 to A5. Therefore, in view of increasing the embossed section joining strengths in dry and wet states, it is advantageous to set the mass mixing ratio of conjugate fibers A to pulp to 1/9 or more, and thereby an embossed section joining strength in dry state of 1.53 N/25 mm or more and an embossed section joining strength in wet state of 0.95 N/ or more can be achieved.

Also, comparison between absorbent articles (i.e., A2 and B2, A3 and B3, A4 and B4, A5 and B5) having the same mass mixing ratio of conjugate fibers A, B to pulpshows that absorbent cores A have embossed section joining strengths in dry and wet states higher than those of absorbent cores B at any mass mixing ratio. Therefore, when the mass mixing ratio of conjugate fibers A to pulp is the same as the mass mixing ratio of conjugate fibers B to pulp, the embossed section joining strengths in dry and wet states can be increased in case where conjugate fibers A are used, as compared with the case where conjugate fibers B are used. In addition, certain embossed section joining strengths in dry and wet states are desired to achieve, the mass mixing ratio of conjugate fiber to pulp can be reduced in case where conjugate fibers A are used, as compared with the case where conjugate fibers B are used (i.e., the mass mixing ratio is increased accordingly, thereby improving the absorbing performance).

As shown in Experimental Examples I-1 and I-2, a fiber falling-off rate in dry state of 2.8% or less, a fiber falling-off rate in wet state of 13.3% or less, an embossed section joining strength in dry state of 1.53 N/25 mm or more, and an embossed section joining strength in wet state of 0.95 N/25 mm or more can be achieved by setting the mass mixing ratio of conjugate fiber to pulp to 1:9 or more, and therefore it is possible to effectively prevent fibers from falling-off from an absorbent core in dry and wet states.

[Experimental Example I-3] Measurements on the maximum tensile strength for absorbent cores A (A2 to A8), B (B1 to B8)

(1) Measurement Method

Absorbent cores A2 to A8, B1 to B8 produced in Production Example I-1 were cut to prepare five sample pieces (150 mm length × 25 mm width), and the sample pieces were used to measure the maximum tensile strength.

[Maximum tensile strength in dry state (N/25 mm)]

A sample piece under standard condition (under an atmosphere of a temperature of 20°C and a humidity of 60%) was attached to a tensile tester (AG-1kNI manufactured by Shimadzu Corporation) at a clamping interval of 100 mm and was loaded at a tensile rate of 100 mm/min. until the sample piece is ruptured (the maximum load) to determine the maximum tensile strength (N) per 25 mm width in the lengthwise direction of the sample piece.

[Maximum tensile strength in wet state (N/25 mm)]

After immersing a sample piece in ion-exchanged water until it is settled down by its own weight or after sinking the sample piece in the water for 1 hour, the maximum tensile strength (N) per 25 mm width in the lengthwise direction of the sample piece was measured in the same manner as described above.

Regarding the measurements of the maximum strengths in dry and wet states, the measurement conditions described in ISO 9073-3 or JIS L 1913 6.3 are used for the measurement conditions other than those specified above.

(2) Results and observation

The measurement results will be shown in Table 5.

[Table 5]

| | | Composition | Maximum tensile strength (N/25 mm) | | | |
|---|---|---|---|---|---|---|
| | | Mass mixing ratio (Pulp : Conjugate fibers) | In dry state | In wet state | Difference between in dry state and in wet state | WEB condition |
| Absorbent cores | A2 | 9:1 | 3.42 | 2.02 | 1.4 | 0.049 |
| | A3 | 8:2 | 9.59 | 5.10 | 4.49 | 0.043 |
| | A4 | 6.5:3.5 | 20.80 | 15.09 | 5.72 | 0.055 |
| | A5 | 5:5 | 40.89 | 33.72 | 7.17 | 0.153 |
| | A6 | 3.5:6.5 | 67.47 | 54.39 | 13.09 | 0.140 |
| | A7 | 2:8 | 83.19 | 81.25 | 1.94 | 0.095 |
| | A8 | 0:10 | 133.64 | 129.06 | 4.58 | 0.060 |
| | B1 | 10:0 | 0.375 | 0.01 | 0.37 | 0.375 |
| | B2 | 9:1 | 0.46 | 0.35 | 0.11 | 0.032 |
| | B3 | 8:2 | 2.33 | 2.04 | 0.30 | 0.190 |
| | B4 | 6.5:3.5 | 7.58 | 6.69 | 0.89 | 0.150 |
| | B5 | 5:5 | 15.70 | 14.70 | 1.00 | 0.025 |
| | B6 | 3.5:6.5 | 27.89 | 25.62 | 2.27 | 0.033 |
| | B7 | 2:8 | 41.52 | 37.88 | 3.64 | 0.017 |
| | B8 | 0:10 | 67.23 | 61.76 | 5.47 | 0.038 |

Observation based on Table 5 is as follows. It is estimated that, in absorbent cores A, a mass mixing ratio of conjugate fibers A to pulp (conjugate fibers A/pulp) of less than 1/9 leads to a maximum tensile strength in wet state of less than 2 N/25 mm, and accordingly it is thought that the strength in wet state cannot be ensured. Therefore, it is thought that absorbent cores A are required to have a mass mixing ratio of conjugate fibers A to pulp of 1/9 or more, in view of retaining strength.

It is estimated that, in absorbent cores B, a mass mixing ratio of conjugate fibers B to pulp (conjugate fibers B/pulp) of less than 2/8 leads to a maximum tensile strength in wet state of less than 2 N/25 mm, and accordingly it is thought that the strength in wet state cannot be ensured. Therefore, it is thought that absorbent cores B are required to have a mass mixing ratio of conjugate fibers B to pulp of 2/8 or more, in view of retaining strength.

Comparison between absorbent cores (for example, A2 and B2) having the same mass mixing ratio of conjugate fibers A, B to pulp shows that absorbent cores A have the maximum tensile strengths (in dry state and wet state) higher than those of absorbent cores B at any mass mixing ratio. In addition, if the mass mixing ratio of conjugate fibers A, B to pulp is in the range of from 1/9 to 6.5/3.5 (absorbent cores A2 to A6, B2 to B6), the differences between the maximum tensile strength in dry state and the maximum tensile strength in wet state (the maximum tensile strength in dry state minus the maximum tensile strength in wet state) of absorbent cores A are greater than those of absorbent cores B.

It is thought that such differences in the strengths are caused by the presence of the hydrogen bonds formed between the oxygen atoms of acyl groups and ether bonds of maleic anhydride and the OH group in the cellulose, whereas such hydrogen bonds are not formed in absorbent core B.

This is also evidenced by the maximum tensile strengths of the samples in the form of web. The maximum tensile strength was measured for the samples in the form of web and was determined to be 0.4 N/25 mm in every samples (see Table 5), suggesting that the difference in the strength is not due to the difference in the degree of entanglement but is due to whether or not hydrogen bonds are formed. The samples in the form of web are samples that have not been subjected to any treatment after stacking a fiber material onto a substrate, or any treatment including entangling treatments such as needle punching; heat treatment with hot air, emboss, energy wave, etc., treatment with an adhesive, etc.

Further, as shown in Table 6, conjugate fibers A has a quantity of heat of fusion greater than that of conjugate fibers B, and therefore it is thought that conjugate fibers A has a crystallinity higher than that of conjugate fibers B, and the difference in strength is also due to the difference in crystallinity (joining strength of the fibers) between conjugate fibers A and B.

[Table 6]

| | | 1st heating | | |
|---|---|---|---|---|
| | | Tim (°C) | Tpm (°C) | ΔH (J/g) |
| Heat fusible conjugate fibers | A | 128/213.6 | 131.0/200.3 | 125.7/34.3 |
| | B | 125.6/249.0 | 128.3/251.4 | 86.9/27.6 |

| | | 2nd heating | | |
|---|---|---|---|---|
| | | Tim (°C) | Tpm (°C) | ΔH (J/g) |
| Heat fusible conjugate fibers | A | 123.9/239.2 | 129.8/254.6 | 129.7/28.1 |
| | B | 122.8/241.8 | 129.1/253.6 | 96.5/18.4 |

Incidentally, Japanese Unexamined Patent Publication No. 2004-270041 describes that a modified polyolefin having maleic anhydride grafted-polymerized thereto have good adhesive properties to cellulose fibers, since the carboxylic anhydride group of the maleic anhydride can be cleaved, thereby forming a covalent bond with a hydroxide group on the surface of cellulose fibers. However, the increase in strength due to the formation of covalent bonds was not observed.

[Production Example I-3] Productions of densified absorbent cores A (A2 to A8), B (B1 to B8)

Densified absorbent cores A2 to A8 (120 mm × 120 mm, each 3 sheets) were produced by placing fiber materials A2 to A8 (see Production Example I-1) on a respective carrier sheet (manufactured by UCKN Co., tissue basis weight: 14 g/m$^2$), bonding them together and thermally fusing conjugate fibers A by means of a common through-air method (heating temperature: 135°C, air flow: 5 m/sec., heating time: 20 sec.), and subsequently adjusting the density to about 0.08 g/cm$^3$ (0.0793 to 0.0817 g/cm$^3$) with a steam jet (SJ) belt press machine.

Densified absorbent cores B1 to B8 (120 mm × 120 mm, each 3 sheets) were produced in the same manner using fiber materials B1 to B8 (see Production Example I-1).

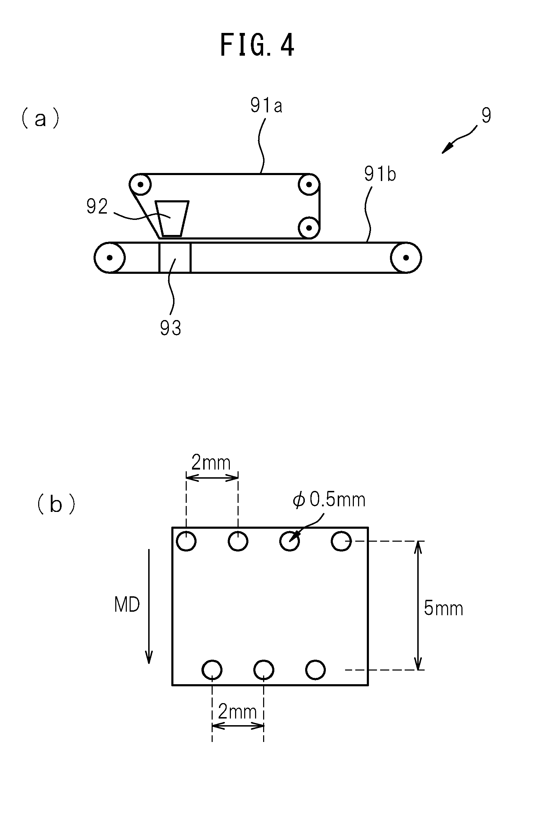

Fig. 4 shows the configuration of the SJ belt press machine used.

As shown in Fig. 4 (a), SJ belt press machine 9 comprises mesh conveyor belts 91a, 91b, steam nozzle 92 and suction box 93, and the absorbent core held between a pair of mesh conveyor belts 91a, 91b is transported between steam nozzle 92 and suction box 93 facing with each other and a high-pressure steam is jetted from steam nozzle 92 to the absorbent core to compress the absorbent core. The steam passed through the absorbent core is sucked by suction box 93 and is discharged. The adjustment of the thickness of the absorbent core can be implemented by the adjustment of the distance between the pair of mesh conveyor belts 91a, 91b.

Mesh conveyor belts 91a, 91b were poly(phenylene sulfide) plain weave mesh conveyors (manufactured by Nippon Filcon Co., Ltd.) and had a wire diameter in vertical and horizontal directions of 0.37 mm, 34 vertical wires per inch, and 32 horizontal wires per inch. The distance between mesh conveyor belts 91a and 91b had been adjusted to 1 mm or 0.2 mm, and the line speed was 200 m/sec.

Steam nozzle 92 had a plurality of openings having a diameter of 0.5 mm, formed at opening pitches of 2 mm and 5 mm, as shown in Fig. 4 (b), and the steam jetted therethrough had a steam pressure of 0.7 MPa and the steam processing amount per unit area was 1.27 $kg/m^2$.

The composition and physical properties of densified absorbent cores A2 to A8 and B1 to B8 will be shown in Table 7.

[Table 7]

| | | Composition | Physical properties after density adjustment | | |
|---|---|---|---|---|---|
| | | Mass mixing ratio (Pulp : Conjugate fibers) | Actual basis weight ($g/m^2$) | Thickness (mm) | Density |
| Densified absorbent cores | A2 | 9:1 | 210 | 2.63 | 0.0798 |
| | A3 | 8:2 | 197 | 2.46 | 0.0801 |
| | A4 | 6.5:3.5 | 206 | 2.55 | 0.0808 |
| | A5 | 5:5 | 215 | 2.71 | 0.0793 |
| | A6 | 3.5:6.5 | 219 | 2.74 | 0.0799 |
| | A7 | 2:8 | 223 | 2.79 | 0.0799 |
| | A8 | 0:10 | 222 | 2.77 | 0.0801 |
| | B1 | 10:0 | - | - | - |
| | B2 | 9:1 | 205 | 2.54 | 0.0807 |
| | B3 | 8:2 | 206 | 2.52 | 0.0817 |
| | B4 | 6.5:3.5 | 215 | 2.63 | 0.0817 |
| | B5 | 5:5 | 214 | 2.63 | 0.0814 |
| | B6 | 3.5:6.5 | 222 | 2.73 | 0.0813 |
| | B7 | 2:8 | 223 | 2.76 | 0.0808 |
| | B8 | 0:10 | 218 | 2.74 | 0.0796 |

[Experimental Example I-4] Measurements on absorbing properties and maximum tensile strength of densified absorbent cores A (A2 to A8), B (B1 to B8)

(1) Measurement methods

[Measurements on absorbing properties (penetration time, liquid drainage time)]

A top sheet (the top sheet of the product of trade name Sophie Hadaomoi) was placed on densified absorbent cores A2 to A8, B1 to B8, and then a holed acrylic plate (a hole of 40 mm × 10 mm at the center, 200 mm (length) × 100 mm (width)) was superposed on the top sheet. Three milliliters of an artificial menstrual blood (prepared by adding 80 g of glycerin, 8 g of sodium carboxymethyl cellulose, 10 g of sodium chloride, 4 g of sodium hydrogen carbonate, 8 g of Red Dye No. 102, 2g of Red Dye No. 2, and 2 g of Yellow Dye No. 5 to 1L of deionized water and stirring them sufficiently) was injected toward the hole in the acrylic plate using an autoburette (Shibata Chemical Instruments Industries Co., Multidosimat E725-1 model) at 90 ml/min. The time after the start of the injection until the artificial menstrual blood retained in the hole in the acrylic plate disappears was determined as a permeation time (sec.), and the time after the start of the injection until the artificial menstrual blood disappears from within the top sheet was determined as drainage time (sec.).

[Measurements on maximum tensile strengths in dry and wet states]

The maximum tensile strengths in dry and wet states were measured for densified absorbent cores A2 to A8, B1 to B8 in the same manner as described for Experimental Example I-3.

(2) Results and observation

The measurement results will be shown in Table 8.

[Table 8]

| | | Composition | Maximum tensile strength (N/25 mm) | | | Penetration rate (sec.) | Surface drainage rate (sec.) |
|---|---|---|---|---|---|---|---|
| | | Mass mixing ratio (Pulp : Conjugate fibers) | In wet state | In wet state | Difference between in dry state and in wet state | | |
| Densified absorbent cores | A2 | 9:1 | 3.69 | 2.17 | 1.52 | 4.54 | 15.34 |
| | A3 | 8:2 | 8.9 | 5.77 | 3.13 | 4.84 | 17.22 |
| | A4 | 6.5:3.5 | 19.37 | 14.75 | 4.62 | 5.52 | 17.95 |
| | A5 | 5:5 | 35.59 | 31.03 | 4.56 | 5.27 | 31.18 |
| | A6 | 3.5:6.5 | 63.22 | 49.36 | 13.86 | 7.73 | 300 ≤ |
| | A7 | 2:8 | 81.11 | 79.36 | 1.75 | 10.03 | 300 ≤ |
| | A8 | 0:10 | 130.39 | 125.94 | 4.45 | 10.33 | 300 ≤ |
| | B1 | 10:0 | 0.375 | 0.01 | 0.37 | - | - |
| | B2 | 9:1 | 0.77 | 0.57 | 0.20 | 4.59 | 16.87 |
| | B3 | 8:2 | 3.67 | 3.08 | 0.59 | 4.89 | 30.98 |
| | B4 | 6.5:3.5 | 8.24 | 7.34 | 0.90 | 5.28 | 40.56 |
| | B5 | 5:5 | 17.6 | 16.3 | 1.30 | 8.25 | 300 ≤ |
| | B6 | 3.5:6.5 | 29.31 | 27.25 | 2.06 | 9.13 | 300 ≤ |
| | B7 | 2:8 | 43.73 | 40.10 | 3.63 | 9.82 | 300 ≤ |
| | B8 | 0:10 | 68.66 | 62.36 | 6.30 | 4.33 | 14.82 |

Observation based on Table 8 is as follows.

A mass mixing ratio of conjugate fibers A to pulp (conjugate fibers A/pulp) in the range of 1/9 to 5/5 (densified absorbent cores A2 to A5) leads to sufficient absorbing properties, whereas a mass mixing ratio of conjugate fibers A to pulp (fiber composite A/pulp) of 6.5/3.5 or more (densified absorbent cores A6 to A8) leads to significant decrease in absorbing properties.

It is estimated that, in densified absorbent cores A, a mass mixing ratio of conjugate fibers A to pulp (conjugate fibers A/pulp) of less than 1/9 leads to a maximum tensile strength in wet state of less than 2 N/25 mm, and accordingly it is thought that the strength in wet state cannot be ensured. Therefore, it is thought that absorbent cores A are required to have a mass mixing ratio of conjugate fibers A to pulp of 1/9 or more, in view of retaining strength.

It is estimated that, in absorbent cores B, a mass mixing ratio of conjugate fibers B to pulp (conjugate fibers B/pulp) of less than 2/8 leads to a maximum tensile strength in wet state of less than 2 N/25 mm, and accordingly it is thought that the strength in wet state cannot be ensured. Therefore, it is thought that absorbent cores B are required to have a mass mixing ratio of conjugate fibers B to pulp (conjugate fibers B/pulp) of 2/8 or more, in view of retaining strength.

When an absorbent core has a density of about 0.08 $g/cm^3$ (0.0793 to 0.0817 $g/cm^3$), a mass mixing ratio of conjugate fibers A to pulp (conjugate fibers A/pulp) in the range of 1/9 to 5/5 allows the absorbent core to have both sufficient strength and absorbing properties. This is because conjugate fibers A can ensure the strength of the absorbent core in a smaller amount than conjugate fibers B (and therefore without inhibiting the absorbing properties).

[Experimental Example I-5]

In Experimental Example I-4, an optimal range of the mass mixing ratio of conjugate fibers A to pulp was investigated for systems in which the density was set to about 0.08 $g/cm^3$ (0.0793 to 0.817 $g/cm^3$), in view of strength and absorbing properties.

In this experimental example, an optimal range of density was investigated in view of absorbing properties.

Densified absorbent cores 1 to 9 having different densities (0.05, 0.06, 0.07, 0.08, 0.09, 0.1, 0.12, 0.13 and 0.14 g/cm$^3$) were produced in the same manner as described for Production Example I-3, using those (basis weight: 200 g/m$^2$) prepared by mixing and stacking a pulp (NB416 manufactured by Wear Hauser Co.) and conjugate fibers A at weight ratios shown in Table 9, and were measured for absorbing properties (liquid drainage time).

The measurement results will be shown in Table 9.

[Table 9]

| | | Composition | Density (g/cm³) | | | | | | | | |
|---|---|---|---|---|---|---|---|---|---|---|---|
| | | Mass mixing ratio (Pulp : Conjugate fibers A) | 0.05 | 0.06 | 0.07 | 0.08 | 0.09 | 0.1 | 0.12 | 0.13 | 0.14 |
| Densified absorbent cores | 1 | 9:1 | 300 | 32.91 | 21.22 | 16.55 | 16.73 | 16.89 | 19.64 | 25 | 27 |
| | 2 | 8:2 | 300 | 36.98 | 23.06 | 17.05 | 17.22 | 17.82 | 21.29 | 29 | 32 |
| | 3 | 6.5:3.5 | 300 | 40.75 | 25.31 | 17.58 | 17.95 | 19.45 | 25.28 | 49.3 | 55.19 |
| | 4 | 5:5 | 300 | 50.81 | 35.46 | 30.79 | 31.18 | 45.66 | 58.36 | 70 | 85 |
| | 5 | 4:6 | 300 | 250 | 250 | 250 | 250 | 250 | 250 | 300 | 300 |
| | 6 | 3.5:6.5 | 300 | 300 | 300 | 300 | 300 | 300 | 300 | 300 | 300 |
| | 7 | 2:8 | 300 | 300 | 300 | 300 | 300 | 300 | 300 | 300 | 300 |
| | 8 | 0:10 | 300 | 300 | 300 | 300 | 300 | 300 | 300 | 300 | 300 |
| | 9 | 10:0 | 250 | 20.33 | 15.11 | 14.72 | 14.82 | 15.21 | 14.53 | 13.77 | 15.48 |

Observation based on Table 9 is as follows.

When the mass mixing ratio of conjugate fibers A to the pulp (conjugate fibers A/pulp) is in the range of 1/9 to 5/5, the density range within which a sufficient liquid drainage performance (specifically, liquid drainage time of 90 seconds or less after dropping a 3 cc of the artificial menstrual blood) is provided is 0.06 to 0.14 g / cm3 .

A density of less than 0.06 $g/cm^3$ leads to a liquid drainage time over 90 seconds at any mass mixing ratio. It is thought that, if the density is less than 0.06 $g/cm^3$, capillary force would not act due to large inter-fiber distance.

When the density is more than 0.12 $g/cm^3$, a mass mixing ratio of conjugate fibers A to the pulp (conjugate fibers A/pulp) in the range of 1/9 to 3.5/6.5 leads to a liquid drainage time of 60 seconds or less, whereas the other range leads to a liquid drainage time of more than 60 seconds. It is thought that, when the density is more than 0.12 $g/cm^3$, although capillary action would act, the space within which a liquid is movable is reduced and thereby the resistance against the movement of the liquid is increased, and therefore the liquid drainage performance is degraded.

From the above experimental examples, it is thought that the optimum range of the mass mixing ratio of conjugate fibers A to the pulp (conjugate fibers A/pulp) is 1/9 to 5/5, and the optimal density is 0.06 to 0.14 $g/cm^3$.

[Production Example II-1] Productions of absorbent cores C (C1 to C5), D (D1 to D6)

(1) Production of core materials C (C1 to C5)

Core materials C1 to C5 (basis weight 200 $g/m^2$) were produced by mixing and stacking a pulp (NB416 manufactured by Wear Hauser Co.), conjugate fibers A and SAP particles at weight ratios of pulp : conjugate fibers A : SAP particles = 9:1:10 (C1), 8:2:10 (C2), 6.5:3.5:10 (C3), 5:5:10 (C4), and 3.5:6.5:10 (C5).

(2) Productions of core materials D (D1 to D6)

Core materials D1 to D6 (basis weight 200 $g/m^2$) were produced by mixing and stacking a pulp (NB416 manufactured by Wear Hauser Co.), conjugate fibers B and SAP particles at weight ratios of pulp : conjugate fibers B : SAP particles = 9:1:10 (D1), 8:2:10 (D2), 6.5:3.5:10 (D3), 5:5:10 (D4), 3.5:6.5:10 (D5), and 10:0:10 (D6).

(3) Productions of absorbent cores C (C1 to C5), D (D1 to D6)

Absorbent cores C1 to C5, D1 to D6 were produced by bonding core materials C1 to C5, D1 to D6 by means of a common through-air method, thermally fusing conjugate fibers A or B. In this case, the heating temperature was set to 135°C, the air flow was set to 5 m/sec, the heating time was set to 20 seconds.

The composition and physical properties of absorbent cores C1 to C5, D1 to D6 will be shown in Table 10.

[Table 10]

| | | Composition | | | | Physical properties after heat treatment | | |
|---|---|---|---|---|---|---|---|---|
| | | Pulp basis weight (g/m$^2$) | Conjugate fiber Basis weight (g/m$^2$) | SAP basis weight (g/m$^2$) | Mass mixing ratio (Pulp : Conjugate fibers : SAP) | Actual basis weight (g/m$^2$) | Thickness (mm) | Density (g/cm$^3$) |
| Absorbent cores | C1 | 90 | 10 | 100 | 9:1:10 | 215 | 3.32 | 0.0648 |
| | C2 | 80 | 20 | 100 | 8:2:10 | 236 | 3.74 | 0.0631 |
| | C3 | 65 | 35 | 100 | 6.5:3.5:10 | 223 | 4.50 | 0.0496 |
| | C4 | 50 | 50 | 100 | 5:5:10 | 234 | 4.68 | 0.0500 |
| | C5 | 35 | 65 | 100 | 3.5:6.5:10 | 237 | 4.67 | 0.0507 |
| | D1 | 90 | 10 | 100 | 9:1:10 | 229 | 3.22 | 0.0711 |
| | D2 | 80 | 20 | 100 | 8:2:10 | 254 | 3.51 | 0.0724 |
| | D3 | 65 | 35 | 100 | 6.5:3.5:10 | 236 | 4.19 | 0.0563 |
| | D4 | 50 | 50 | 100 | 5:5:10 | 229 | 4.22 | 0.0543 |
| | D5 | 35 | 65 | 100 | 3.5:6.5:10 | 241 | 4.62 | 0.0522 |
| | D6 | 100 | 0 | 100 | 10:0:10 | 188 | 2.71 | 0.0694 |

[Experimental Example II-1] Measurements on SAP particles falling-off rate in dry state for absorbent cores C (C1 to C5), D (D1 to D6)

(1) Measurement Method

Absorbent cores C1 to C5, D1 to D6 produced in Production Example II-1 were cut to prepare sample pieces (100 mm length × 100 mm width), and the resulting sample pieces were used for the measurement on SAP particles falling-off rate (%) in dry state.

[SAP particle falling-off rate in dry state (%)]

An empty 2000 mL vessel (JP-2000 manufactured by NIKKO Co., Ltd.) was charged with pre-test sample pieces (100 mm × 100 mm) and was shaken with a shaker (SHKV-200 manufactured by IWAKI, Co., Ltd.) at a shaking rate of 300 rpm for 10 minutes, and a sample piece which maintained a sheet form after shaking was removed from the vessel and was marked as a post-test sample piece. Then, a SAP particle falling-off rate (%) (the weight of the fallen SAP particles/the weight of the pre-test sample piece × 100) was calculated based on the weight of the fallen SAP particles (the weight of the pre-test sample piece minus the weight of the post-test sample piece).

(2) Results and observation

The measurement results will be shown in Table 11.

[Table 11]

| | | Composition | In dry state | | | |
|---|---|---|---|---|---|---|
| | | Mass mixing ratio (Pulp : Conjugate fibers : SAP) | Pre-test weight (g) | Post-test weight (g) | Weight of fallen SAP (g) | SAP particles falling-off rate (%) |
| Absorbent cores | C1 | 9:1:10 | 2.10 | 1.95 | 0.30 | 14.31 |
| | C2 | 8:2:10 | 2.17 | 2.06 | 0.22 | 10.29 |
| | C3 | 6.5:3.5:10 | 2.09 | 1.96 | 0.13 | 6.20 |
| | C4 | 5:5:10 | 2.16 | 2.08 | 0.08 | 3.70 |
| | C5 | 3.5:6.5:10 | 2.18 | 2.11 | 0.07 | 3.30 |
| | D1 | 9:1:10 | 2.10 | 1.63 | 0.47 | 22.33 |
| | D2 | 8:2:10 | 2.10 | 1.66 | 0.44 | 20.95 |
| | D3 | 6.5:3.5:10 | 2.07 | 1.75 | 0.32 | 15.31 |
| | D4 | 5:5:10 | 2.12 | 1.85 | 0.27 | 12.74 |
| | D5 | 3.5:6.5:10 | 2.24 | 2.14 | 0.11 | 4.69 |
| | D6 | 10:0:10 | 2.15 | 0.22 | 1.93 | 89.77 |

Observation based on Table 11 is as follows.

Comparison among absorbent cores C1 to C5 shows that the greater the mass mixing ratio of conjugate fibers A to pulp (conjugate fibers A/pulp), the lower the SAP particle falling-off rate in dry state. Therefore, in view of decreasing the SAP particle falling-off rate in dry state, it is advantageous to set the mass mixing ratio of conjugate fibers A to pulp to 1/9 or more, and thereby a SAP particle falling-off rate in dry state of 14.3% or less can be achieved. In addition, it is thought that, if the mass mixing ratio of conjugate fibers A to pulp is less than 1/9, the fiber number of conjugate fibers A is too low, and therefore a network of fibers (between conjugate fibers A, between conjugate fibers A and pulp) is not sufficiently formed in conjugate fibers A, resulting a large SAP particle falling-off rate in dry state.

Also, comparison between absorbent cores (i.e., C1 and D1, C2 and D2, C3 and D3, C4 and D4, C5 and D5) having the same mass mixing ratio of conjugate fibers A, B to pulp shows that absorbent cores C have a SAP particle falling-off rate in dry state lower than that of absorbent cores D at any mass mixing ratio. Therefore, when the mass mixing ratio of conjugate fibers A to pulp is the same as the mass mixing ratio of conjugate fibers B to pulp, the SAP particle falling-off rate in dry state can be reduced in case where conjugate fibers A are used, as compared with the case where conjugate fibers B are used. In addition, if a certain SAP particle falling-off rate in dry state is desired to be achieved, the mass mixing ratio of conjugate fibers to pulp can be reduced in case where conjugate fibers A are used, as compared with the case where conjugate fibers B are used. The mass mixing ratio of pulp is increased accordingly, thereby improving the absorbing performance of the absorbent cores.

[Experimental Example II-2] Measurements on maximum tensile strengths for absorbent cores C (C1 to C5), D (D1 to D6)

(1) Measurement Methods

Absorbent cores C1 to C5, D1 to D6 produced in Example II-1 were cut into five sample pieces (150 mm length $\times$ 25 mm width), and were used to measure the maximum tensile strength.

[Maximum tensile strength in dry state (N/25 mm)]

A sample piece under standard condition (under an atmosphere of a temperature of 20°C and a humidity of 60%) was attached to a tensile tester (AG-1kNI manufactured by Shimadzu Corporation) at a clamping interval of 100 mm and was loaded at a tensile rate of 100 mm/min. until the sample piece is ruptured (the maximum load) to determine the maximum tensile strength (N) per 25 mm width in the lengthwise direction (MD direction) of the sample piece.

[Maximum tensile strength in wet state (N/25 mm)]

After immersing a sample piece in ion-exchanged water until it is settled down by its own weight or after sinking the sample piece in the water for 1 hour, the maximum tensile strength (N) per 25 mm width in the lengthwise direction (MD direction) of the sample piece was measured in the same manner as described above.

Regarding the measurements of the maximum strengths in dry and wet states, the measurement conditions described in ISO 9073-3 or JIS L 1913 6.3 are used for the measurement conditions other than those specified above.

(2) Results and observation

The measurement results will be shown in Table 12.

[Table 12]

| | | Composition | Maximum tensile strength (N/25 mm) | | |
|---|---|---|---|---|---|
| | | Mass mixing ratio (Pulp : Conjugate fibers : SAP) | In dry state | In wet state | Difference between in dry state and in wet state |
| Absorbent cores | C1 | 9:1:10 | 1.05 | 0.90 | 0.15 |
| | C2 | 8:2:10 | 4.20 | 2.89 | 1.31 |
| | C3 | 6.5:3.5:10 | 9.61 | 8.76 | 0.85 |
| | C4 | 5:5:10 | 17.20 | 15.63 | 1.57 |
| | C5 | 3.5:6.5:10 | 28.00 | 25.40 | 2.60 |
| | D1 | 9:1:10 | 0.56 | 0.55 | 0.01 |
| | D2 | 8:2:10 | 1.60 | 1.42 | 0.18 |
| | D3 | 6.5:3.5:10 | 5.67 | 4.13 | 1.53 |
| | D4 | 5:5:10 | 11.26 | 10.96 | 0.30 |
| | D5 | 3.5:6.5:10 | 16.30 | 15.28 | 1.02 |
| | D6 | 10:0:10 | 0.09 | 0.08 | 0.01 |

Observation based on Table 12 is as follows.

Comparison between absorbent cores (i.e., C1 and D1, C2 and D2, C3 and D3, C4 and D4, C5 and D5) having the same mass mixing ratio of conjugate fibers A, B to pulp shows that absorbent cores C have the maximum tensile strengths (in dry and wet states) greater than those of absorbent cores D at any mass mixing ratio.

It is thought that such differences in the strengths are resulted from the presence of the hydrogen bonds in absorbent cores C, which were formed between the oxygen atoms of acyl groups and ether bonds in maleic anhydride and the OH group in the cellulose, whereas such hydrogen bonds were not formed in absorbent core D. In addition, cit is thought that conjugate fibers A has a crystallinity higher than that of conjugate fibers B, and the differences in the strengths are also due to the difference in crystallinity (joining strength of the fibers) between conjugate fibers A and B. For the details, see Experimental Example I-3.

Explanation of Symbols

1    Sanitary napkin (absorbent article)
2    Top sheet (liquid-permeable sheet)
3    Back sheet (liquid impermeable sheet)
4    Absorbent core

**Claims**

1. An absorbent article comprising a liquid-permeable layer, a liquid-impermeable layer, and an absorbent core disposed between the liquid-permeable layer and the liquid-impermeable layer,
wherein the absorbent core comprises as constituent fibers cellulose-based water absorbent fibers and thermoplastic resin fibers comprising as a monomer component a unsaturated carboxylic acid, a unsaturated carboxylic acid anhydride, or a mixture thereof,
wherein the absorbent core has a non-covered region where the constituent fibers thereof are exposed to a surface so that the constituent fibers directly contact with the liquid-permeable layer,
wherein the weight ratio of the thermoplastic resin fibers to the water absorbent fibers in the absorbent core is 1/9 or more, and
wherein the absorbent core has a fiber falling-off rate in wet state of 13.3% or less.

2. The absorbent article of claim 1, wherein the absorbent core has a fiber falling-off rate in dry state of 2.8% or less.

3. The absorbent article of claim 1 or 2, wherein the absorbent core comprises super water absorbent resin (SAP) particles, and the absorbent core has a SAP particle falling-off rate in dry state of 14.3% or less.

4. The absorbent article of any one of claims 1 to 3, wherein the weight ratio of the thermoplastic resin fibers to the water absorbent fibers in the absorbent core is 1/9 to 5/5.

5. The absorbent article of claim 4, wherein the absorbent core has a fiber falling-off rate in dry state of 0.9 to 2.8%, and the absorbent core has a fiber falling-off rate in wet state of 1.0 to 13.3%.

6. The absorbent article of claim 4 or 5, wherein the absorbent core has a fiber density of 0.06 to 0.14 $g/cm^3$.

7. The absorbent article of claim 6, wherein the absorbent core is obtained by ejecting a high-pressure steam to a mixed material comprising the cellulose-based water absorbent fibers and the thermoplastic resin fibers to densify the mixed material.

8. The absorbent article of claim 6 or 7, wherein the absorbent core has a fiber basis weight of 40 to 900 $g/m^2$.

9. The absorbent article of any one of claims 4 to 8, further comprising a joint section at which the liquid-permeable layer and the absorbent core are joined with each other, wherein the joint section has a joining strength in dry state of 1.53 to 7.65 N/25 mm, and the joint section has a joining strength in wet state of 0.95 to 4.34 N/25 mm.

10. The absorbent article of claim 9, wherein the joint section is a compressed section which integrates the liquid-permeable layer with the absorbent in the thickness direction.

11. The absorbent article of any one of claims 1 to 10, wherein the constituent fibers of the absorbent core are adhered to each other.

12. The absorbent article of any one of claims 1 to 11, wherein the liquid-permeable layer has through holes formed therethrough at an opening ratio of 5 to 70%.

13. The absorbent article of any one of claims 1 to 12, wherein the liquid-permeable layer and the absorbent core have through-holes formed therethrough.

14. The absorbent article of any one of claims 1 to 13, wherein the thermoplastic resin fibers are core-sheath type conjugate fibers comprising as a sheath component a modified polyolefin having a vinyl monomer graft-polymerized thereto, or a mixed polymer of the modified polyolefin and other resins, and as a core component a resin having a melting point higher than that of the modified polyolefin, wherein the vinyl monomer comprises a unsaturated carboxylic acid, a unsaturated carboxylic acid anhydride, or a mixture thereof.

15. The absorbent article of any one of claims 1 to 14, wherein the unsaturated carboxylic acid, unsaturated carboxylic acid anhydride, or a mixture thereof is maleic acid or a derivative thereof, maleic anhydride or a derivative thereof, or a mixture thereof.

**16.** The absorbent article of any one of claims 1 to 15, wherein the thermoplastic resin fiber included in the absorbent core is colored.

# FIG. 1

FIG. 2

# FIG. 3

4th Step     3rd Step     2nd Step     1st Step

EP 2 901 984 A1

# FIG. 4

(a)

(b)

## INTERNATIONAL SEARCH REPORT

| International application No. |
| --- |
| PCT/JP2013/072161 |

### A. CLASSIFICATION OF SUBJECT MATTER
*A61F13/15*(2006.01)i, *A61F13/53*(2006.01)i

According to International Patent Classification (IPC) or to both national classification and IPC

### B. FIELDS SEARCHED

Minimum documentation searched (classification system followed by classification symbols)
A61F13/00, A61F13/15-13/84

Documentation searched other than minimum documentation to the extent that such documents are included in the fields searched

| | | | |
| --- | --- | --- | --- |
| Jitsuyo Shinan Koho | 1922-1996 | Jitsuyo Shinan Toroku Koho | 1996-2013 |
| Kokai Jitsuyo Shinan Koho | 1971-2013 | Toroku Jitsuyo Shinan Koho | 1994-2013 |

Electronic data base consulted during the international search (name of data base and, where practicable, search terms used)

### C. DOCUMENTS CONSIDERED TO BE RELEVANT

| Category* | Citation of document, with indication, where appropriate, of the relevant passages | Relevant to claim No. |
| --- | --- | --- |
| Y | JP 2005-538772 A (The Procter & Gamble Co.), 22 December 2005 (22.12.2005), paragraphs [0005] to [0008], [0019] to [0025], [0051] to [0063], [0071]; fig. 1 to 4 & EP 1536747 A1 & WO 2004/024044 A1 & CN 1678262 A & AU 2003272335 A1 | 1-6,8-16 |
| Y A | JP 2005-095481 A (Chisso Corp.), 14 April 2005 (14.04.2005), paragraphs [0007] to [0046], [0058], [0069], [0070]; fig. 1 (Family: none) | 1-6,8-16 7 |
| Y | JP 2002-119539 A (Zuiko Corp.), 23 April 2002 (23.04.2002), paragraph [0022]; fig. 3 (Family: none) | 13 |

☒ Further documents are listed in the continuation of Box C. ☐ See patent family annex.

| | | | |
| --- | --- | --- | --- |
| * | Special categories of cited documents: | "T" | later document published after the international filing date or priority date and not in conflict with the application but cited to understand the principle or theory underlying the invention |
| "A" | document defining the general state of the art which is not considered to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
| --- | --- |
| 07 October, 2013 (07.10.13) | 15 October, 2013 (15.10.13) |

| Name and mailing address of the ISA/ Japanese Patent Office | Authorized officer |
| --- | --- |
| Facsimile No. | Telephone No. |

Form PCT/ISA/210 (second sheet) (July 2009)

**INTERNATIONAL SEARCH REPORT**

| International application No. |
| --- |
| PCT/JP2013/072161 |

C (Continuation). DOCUMENTS CONSIDERED TO BE RELEVANT

| Category* | Citation of document, with indication, where appropriate, of the relevant passages | Relevant to claim No. |
| --- | --- | --- |
| Y | JP 2004-187732 A (Uni-Charm Corp.),<br>08 July 2004 (08.07.2004),<br>paragraph [0051]; fig. 1, 3<br>& KR 10-2004-0049812 A & CN 1507844 A | 16 |

Form PCT/ISA/210 (continuation of second sheet) (July 2009)

**EP 2 901 984 A1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2012075637 A
- JP 4221849 B
- JP 2004270041 A